# EUROPEAN PATENT APPLICATION

(11) **EP 3 875 111 A1**
(43) Date of publication of application: **08.09.2021**
(21) Application number: 20461518.1
(22) Date of filing: 05.03.2020
(51) Int. Cl.: A61K 39/00, A61P 35/00

(54) **STEM CELL CANCER VACCINE COMPOSITION**

(71) Applicant: Poznan University Of Medical Sciences, 61-701 Poznan (PL); Mackiewicz, Andrzej, 61-051 Poznan (PL)
(72) Inventor: Mackiewicz, Andrzej, 61-051 Pozna (PL)
(74) Representative: Zwicker, Jörk

(57) **Abstract**

The invention relates to tumour therapy. In particular, the present invention relates to cancer vaccine compositions comprising both allogenic cancer cells modified with hyper-cytokines and stem cells. The invention further relates to a pharmaceutical composition comprising the cancer vaccine composition and to the cancer vaccine composition for use in a method of treatment of cancer, prevention of recurrence of cancer and/or prevention of cancer in general and in particular for the treatment, prevention of recurrence and prevention of melanoma.

SEQUENCE LISTING

<110>  Poznan University of Medical Sciences, et al.

<120>  STEM CELL CANCER VACCINE COMPOSITION

<130>  884-1 EP

<160>  15

<170>  PatentIn version 3.5

<210>  1
<211>  468
<212>  PRT
<213>  Homo sapiens

<400>  1

Met Leu Ala Val Gly Cys Ala Leu Leu Ala Ala Leu Leu Ala Ala Pro
1               5                   10                  15

Gly Ala Ala Leu Ala Pro Arg Arg Cys Pro Ala Gln Glu Val Ala Arg
            20                  25                  30

Gly Val Leu Thr Ser Leu Pro Gly Asp Ser Val Thr Leu Thr Cys Pro
        35                  40                  45

Gly Val Glu Pro Glu Asp Asn Ala Thr Val His Trp Val Leu Arg Lys
    50                  55                  60

Pro Ala Ala Gly Ser His Pro Ser Arg Trp Ala Gly Met Gly Arg Arg
65                  70                  75                  80

Leu Leu Leu Arg Ser Val Gln Leu His Asp Ser Gly Asn Tyr Ser Cys
                85                  90                  95

Tyr Arg Ala Gly Arg Pro Ala Gly Thr Val His Leu Leu Val Asp Val
            100                 105                 110

Pro Pro Glu Glu Pro Gln Leu Ser Cys Phe Arg Lys Ser Pro Leu Ser
        115                 120                 125

Asn Val Val Cys Glu Trp Gly Pro Arg Ser Thr Pro Ser Leu Thr Thr
    130                 135                 140

Lys Ala Val Leu Leu Val Arg Lys Phe Gln Asn Ser Pro Ala Glu Asp
145                 150                 155                 160

Phe Gln Glu Pro Cys Gln Tyr Ser Gln Glu Ser Gln Lys Phe Ser Cys
                165                 170                 175

Gln Leu Ala Val Pro Glu Gly Asp Ser Ser Phe Tyr Ile Val Ser Met
        180                 185                 190

Cys Val Ala Ser Ser Val Gly Ser Lys Phe Ser Lys Thr Gln Thr Phe
    195                 200                 205

Gln Gly Cys Gly Ile Leu Gln Pro Asp Pro Pro Ala Asn Ile Thr Val
210                 215                 220

Thr Ala Val Ala Arg Asn Pro Arg Trp Leu Ser Val Thr Trp Gln Asp
225                 230                 235                 240

Pro His Ser Trp Asn Ser Ser Phe Tyr Arg Leu Arg Phe Glu Leu Arg
                245                 250                 255

Tyr Arg Ala Glu Arg Ser Lys Thr Phe Thr Thr Trp Met Val Lys Asp
            260                 265                 270

Leu Gln His His Cys Val Ile His Asp Ala Trp Ser Gly Leu Arg His
        275                 280                 285

Val Val Gln Leu Arg Ala Gln Glu Glu Phe Gly Gln Gly Glu Trp Ser
    290                 295                 300

Glu Trp Ser Pro Glu Ala Met Gly Thr Pro Trp Thr Glu Ser Arg Ser
305                 310                 315                 320

Pro Pro Ala Glu Asn Glu Val Ser Thr Pro Met Gln Ala Leu Thr Thr
                325                 330                 335

Asn Lys Asp Asp Asp Asn Ile Leu Phe Arg Asp Ser Ala Asn Ala Thr
            340                 345                 350

Ser Leu Pro Val Gln Asp Ser Ser Ser Val Pro Leu Pro Thr Phe Leu
        355                 360                 365

Val Ala Gly Gly Ser Leu Ala Phe Gly Thr Leu Leu Cys Ile Ala Ile
    370                 375                 380

Val Leu Arg Phe Lys Lys Thr Trp Lys Leu Arg Ala Leu Lys Glu Gly
385                 390                 395                 400

Lys Thr Ser Met His Pro Pro Tyr Ser Leu Gly Gln Leu Val Pro Glu
                405                 410                 415

Arg Pro Arg Pro Thr Pro Val Leu Val Pro Leu Ile Ser Pro Pro Val
            420                 425                 430

Ser Pro Ser Ser Leu Gly Ser Asp Asn Thr Ser Ser His Asn Arg Pro
        435                 440                 445

Asp Ala Arg Asp Pro Arg Ser Pro Tyr Asp Ile Ser Asn Thr Asp Tyr
    450                 455                 460

Phe Phe Pro Arg
465

<210>  2
<211>  212
<212>  PRT
<213>  Homo sapiens

<400>  2

Met Asn Ser Phe Ser Thr Ser Ala Phe Gly Pro Val Ala Phe Ser Leu
1               5                   10                  15

Gly Leu Leu Leu Val Leu Pro Ala Ala Phe Pro Ala Pro Val Pro Pro
            20                  25                  30

Gly Glu Asp Ser Lys Asp Val Ala Ala Pro His Arg Gln Pro Leu Thr
        35                  40                  45

Ser Ser Glu Arg Ile Asp Lys Gln Ile Arg Tyr Ile Leu Asp Gly Ile
    50                  55                  60

Ser Ala Leu Arg Lys Glu Thr Cys Asn Lys Ser Asn Met Cys Glu Ser
65                  70                  75                  80

Ser Lys Glu Ala Leu Ala Glu Asn Asn Leu Asn Leu Pro Lys Met Ala
                85                  90                  95

Glu Lys Asp Gly Cys Phe Gln Ser Gly Phe Asn Glu Glu Thr Cys Leu
            100                 105                 110

Val Lys Ile Ile Thr Gly Leu Leu Glu Phe Glu Val Tyr Leu Glu Tyr
        115                 120                 125

Leu Gln Asn Arg Phe Glu Ser Ser Glu Glu Gln Ala Arg Ala Val Gln
    130                 135                 140

Met Ser Thr Lys Val Leu Ile Gln Phe Leu Gln Lys Lys Ala Lys Asn
145                 150                 155                 160

Leu Asp Ala Ile Thr Thr Pro Asp Pro Thr Thr Asn Ala Ser Leu Leu
                165                 170                 175

Thr Lys Leu Gln Ala Gln Asn Gln Trp Leu Gln Asp Met Thr Thr His
        180                 185                 190

Leu Ile Leu Arg Ser Phe Lys Glu Phe Leu Gln Ser Ser Leu Arg Ala
    195                 200                 205

Leu Arg Gln Met
    210

<210>  3
<211>  422
<212>  PRT
<213>  Homo sapiens

<400>  3

Met Gly Ser Ser Cys Ser Gly Leu Ser Arg Val Leu Val Ala Val Ala
1               5                   10                  15

Thr Ala Leu Val Ser Ala Ser Ser Pro Cys Pro Gln Ala Trp Gly Pro
            20                  25                  30

Pro Gly Val Gln Tyr Gly Gln Pro Gly Arg Ser Val Lys Leu Cys Cys
        35                  40                  45

Pro Gly Val Thr Ala Gly Asp Pro Val Ser Trp Phe Arg Asp Gly Glu
    50                  55                  60

Pro Lys Leu Leu Gln Gly Pro Asp Ser Gly Leu Gly His Glu Leu Val
65                  70                  75                  80

Leu Ala Gln Ala Asp Ser Thr Asp Glu Gly Thr Tyr Ile Cys Gln Thr
                85                  90                  95

Leu Asp Gly Ala Leu Gly Gly Thr Val Thr Leu Gln Leu Gly Tyr Pro
            100                 105                 110

Pro Ala Arg Pro Val Val Ser Cys Gln Ala Ala Asp Tyr Glu Asn Phe
        115                 120                 125

Ser Cys Thr Trp Ser Pro Ser Gln Ile Ser Gly Leu Pro Thr Arg Tyr
    130                 135                 140

Leu Thr Ser Tyr Arg Lys Lys Thr Val Leu Gly Ala Asp Ser Gln Arg
145                 150                 155                 160

Arg Ser Pro Ser Thr Gly Pro Trp Pro Cys Pro Gln Asp Pro Leu Gly
                165                 170                 175

Ala Ala Arg Cys Val Val His Gly Ala Glu Phe Trp Ser Gln Tyr Arg
        180                 185                 190

Ile Asn Val Thr Glu Val Asn Pro Leu Gly Ala Ser Thr Arg Leu Leu
    195                 200                 205

Asp Val Ser Leu Gln Ser Ile Leu Arg Pro Asp Pro Pro Gln Gly Leu
    210                 215                 220

Arg Val Glu Ser Val Pro Gly Tyr Pro Arg Arg Leu Arg Ala Ser Trp
225                 230                 235                 240

Thr Tyr Pro Ala Ser Trp Pro Cys Gln Pro His Phe Leu Leu Lys Phe
                245                 250                 255

Arg Leu Gln Tyr Arg Pro Ala Gln His Pro Ala Trp Ser Thr Val Glu
            260                 265                 270

Pro Ala Gly Leu Glu Glu Val Ile Thr Asp Ala Val Ala Gly Leu Pro
        275                 280                 285

His Ala Val Arg Val Ser Ala Arg Asp Phe Leu Asp Ala Gly Thr Trp
    290                 295                 300

Ser Thr Trp Ser Pro Glu Ala Trp Gly Thr Pro Ser Thr Gly Thr Ile
305                 310                 315                 320

Pro Lys Glu Ile Pro Ala Trp Gly Gln Leu His Thr Gln Pro Glu Val
                325                 330                 335

Glu Pro Gln Val Asp Ser Pro Ala Pro Pro Arg Pro Ser Leu Gln Pro
            340                 345                 350

His Pro Arg Leu Leu Asp His Arg Asp Ser Val Glu Gln Val Ala Val
        355                 360                 365

Leu Ala Ser Leu Gly Ile Leu Ser Phe Leu Gly Leu Val Ala Gly Ala
    370                 375                 380

Leu Ala Leu Gly Leu Trp Leu Arg Leu Arg Arg Gly Gly Lys Asp Gly
385                 390                 395                 400

Ser Pro Lys Pro Gly Phe Leu Ala Ser Val Ile Pro Val Asp Arg Arg
                405                 410                 415

Pro Gly Ala Pro Asn Leu
            420

<210>  4
<211>  199
<212>  PRT
<213>  Homo sapiens

<400>  4

Met Asn Cys Val Cys Arg Leu Val Leu Val Val Leu Ser Leu Trp Pro
1               5                   10                  15

Asp Thr Ala Val Ala Pro Gly Pro Pro Pro Gly Pro Pro Arg Val Ser
            20                  25                  30

Pro Asp Pro Arg Ala Glu Leu Asp Ser Thr Val Leu Leu Thr Arg Ser
        35                  40                  45

Leu Leu Ala Asp Thr Arg Gln Leu Ala Ala Gln Leu Arg Asp Lys Phe
    50                  55                  60

Pro Ala Asp Gly Asp His Asn Leu Asp Ser Leu Pro Thr Leu Ala Met
65                  70                  75                  80

Ser Ala Gly Ala Leu Gly Ala Leu Gln Leu Pro Gly Val Leu Thr Arg
                85                  90                  95

Leu Arg Ala Asp Leu Leu Ser Tyr Leu Arg His Val Gln Trp Leu Arg
            100                 105                 110

Arg Ala Gly Gly Ser Ser Leu Lys Thr Leu Glu Pro Glu Leu Gly Thr
        115                 120                 125

Leu Gln Ala Arg Leu Asp Arg Leu Leu Arg Arg Leu Gln Leu Leu Met
    130                 135                 140

Ser Arg Leu Ala Leu Pro Gln Pro Pro Pro Asp Pro Pro Ala Pro Pro
145                 150                 155                 160

Leu Ala Pro Pro Ser Ser Ala Trp Gly Gly Ile Arg Ala Ala His Ala
                165                 170                 175

Ile Leu Gly Gly Leu His Leu Thr Leu Asp Trp Ala Val Arg Gly Leu
            180                 185                 190

Leu Leu Leu Lys Thr Arg Leu
        195

<210>  5
<211>  408

<212>  PRT
<213>  Artificial

<220>
<223>  Hypercytokine H-IL-6, Fischer et al. 1997

<400>  5

Pro Pro Glu Glu Pro Gln Leu Ser Cys Phe Arg Lys Ser Pro Leu Ser
1               5                   10                  15

Asn Val Val Cys Glu Trp Gly Pro Arg Ser Thr Pro Ser Leu Thr Thr
            20                  25                  30

Lys Ala Val Leu Leu Val Arg Lys Phe Gln Asn Ser Pro Ala Glu Asp
        35                  40                  45

Phe Gln Glu Pro Cys Gln Tyr Ser Gln Glu Ser Gln Lys Phe Ser Cys
    50                  55                  60

Gln Leu Ala Val Pro Glu Gly Asp Ser Ser Phe Tyr Ile Val Ser Met
65                  70                  75                  80

Cys Val Ala Ser Ser Val Gly Ser Lys Phe Ser Lys Thr Gln Thr Phe
                85                  90                  95

Gln Gly Cys Gly Ile Leu Gln Pro Asp Pro Pro Ala Asn Ile Thr Val
            100                 105                 110

Thr Ala Val Ala Arg Asn Pro Arg Trp Leu Ser Val Thr Trp Gln Asp
        115                 120                 125

Pro His Ser Trp Asn Ser Ser Phe Tyr Arg Leu Arg Phe Glu Leu Arg
    130                 135                 140

Tyr Arg Ala Glu Arg Ser Lys Thr Phe Thr Thr Trp Met Val Lys Asp
145                 150                 155                 160

Leu Gln His His Cys Val Ile His Asp Ala Trp Ser Gly Leu Arg His
                165                 170                 175

Val Val Gln Leu Arg Ala Gln Glu Glu Phe Gly Gln Gly Glu Trp Ser
            180                 185                 190

Glu Trp Ser Pro Glu Ala Met Gly Thr Pro Trp Thr Glu Ser Arg Ser
        195                 200                 205

Pro Pro Ala Arg Gly Gly Gly Gly Ser Gly Gly Gly Gly Ser Val Glu
    210                 215                 220

Pro Val Pro Pro Gly Glu Asp Ser Lys Asp Val Ala Ala Pro His Arg
225                 230                 235                 240

Gln Pro Leu Thr Ser Ser Glu Arg Ile Asp Lys Gln Ile Arg Tyr Ile
                245                 250                 255

Leu Asp Gly Ile Ser Ala Leu Arg Lys Glu Thr Cys Asn Lys Ser Asn
            260                 265                 270

Met Cys Glu Ser Ser Lys Glu Ala Leu Ala Glu Asn Asn Leu Asn Leu
        275                 280                 285

Pro Lys Met Ala Glu Lys Asp Gly Cys Phe Gln Ser Gly Phe Asn Glu
    290                 295                 300

Glu Thr Cys Leu Val Lys Ile Ile Thr Gly Leu Leu Glu Phe Glu Val
305                 310                 315                 320

Tyr Leu Glu Tyr Leu Gln Asn Arg Phe Glu Ser Ser Glu Glu Gln Ala
                325                 330                 335

Arg Ala Val Gln Met Ser Thr Lys Val Leu Ile Gln Phe Leu Gln Lys
            340                 345                 350

Lys Ala Lys Asn Leu Asp Ala Ile Thr Thr Pro Asp Pro Thr Thr Asn
        355                 360                 365

Ala Ser Leu Leu Thr Lys Leu Gln Ala Gln Asn Gln Trp Leu Gln Asp
    370                 375                 380

Met Thr Thr His Leu Ile Leu Arg Ser Phe Lys Glu Phe Leu Gln Ser
385                 390                 395                 400

Ser Leu Arg Ala Leu Arg Gln Met
                405

<210>  6
<211>  543
<212>  PRT
<213>  Artificial

<220>
<223>  sIL-6R/IL-6 with linker EFM, Chebath et al. 1997

<400>  6

Met Leu Ala Val Gly Cys Ala Leu Leu Ala Ala Leu Leu Ala Ala Pro
1               5                   10                  15

Gly Ala Ala Leu Ala Pro Arg Arg Cys Pro Ala Gln Glu Val Ala Arg
            20                  25                  30

Gly Val Leu Thr Ser Leu Pro Gly Asp Ser Val Thr Leu Thr Cys Pro
        35                  40                  45

Gly Val Glu Pro Glu Asp Asn Ala Thr Val His Trp Val Leu Arg Lys
    50                  55                  60

Pro Ala Ala Gly Ser His Pro Ser Arg Trp Ala Gly Met Gly Arg Arg
65                  70                  75                  80

Leu Leu Leu Arg Ser Val Gln Leu His Asp Ser Gly Asn Tyr Ser Cys
                85                  90                  95

Tyr Arg Ala Gly Arg Pro Ala Gly Thr Val His Leu Leu Val Asp Val
            100                 105                 110

Pro Pro Glu Glu Pro Gln Leu Ser Cys Phe Arg Lys Ser Pro Leu Ser
        115                 120                 125

Asn Val Val Cys Glu Trp Gly Pro Arg Ser Thr Pro Ser Leu Thr Thr
    130                 135                 140

Lys Ala Val Leu Leu Val Arg Lys Phe Gln Asn Ser Pro Ala Glu Asp
145                 150                 155                 160

Phe Gln Glu Pro Cys Gln Tyr Ser Gln Glu Ser Gln Lys Phe Ser Cys
                165                 170                 175

Gln Leu Ala Val Pro Glu Gly Asp Ser Ser Phe Tyr Ile Val Ser Met
            180                 185                 190

Cys Val Ala Ser Ser Val Gly Ser Lys Phe Ser Lys Thr Gln Thr Phe
        195                 200                 205

Gln Gly Cys Gly Ile Leu Gln Pro Asp Pro Pro Ala Asn Ile Thr Val
    210                 215                 220

Thr Ala Val Ala Arg Asn Pro Arg Trp Leu Ser Val Thr Trp Gln Asp
225                 230                 235                 240

Pro His Ser Trp Asn Ser Ser Phe Tyr Arg Leu Arg Phe Glu Leu Arg
                245                 250                 255

Tyr Arg Ala Glu Arg Ser Lys Thr Phe Thr Thr Trp Met Val Lys Asp
            260                 265                 270

Leu Gln His His Cys Val Ile His Asp Ala Trp Ser Gly Leu Arg His
        275                 280                 285

Val Val Gln Leu Arg Ala Gln Glu Glu Phe Gly Gln Gly Glu Trp Ser
    290                 295                 300

Glu Trp Ser Pro Glu Ala Met Gly Thr Pro Trp Thr Glu Ser Arg Ser
305                 310                 315                 320

Pro Pro Ala Glu Asn Glu Val Ser Thr Pro Met Gln Ala Leu Thr Thr
                325                 330                 335

Asn Lys Asp Asp Asp Asn Ile Leu Phe Arg Asp Ser Ala Asn Ala Thr
            340                 345                 350

Ser Leu Pro Val Glu Phe Met Pro Val Pro Pro Gly Glu Asp Ser Lys
        355                 360                 365

Asp Val Ala Ala Pro His Arg Gln Pro Leu Thr Ser Ser Glu Arg Ile
    370                 375                 380

Asp Lys Gln Ile Arg Tyr Ile Leu Asp Gly Ile Ser Ala Leu Arg Lys
385                 390                 395                 400

Glu Thr Cys Asn Lys Ser Asn Met Cys Glu Ser Ser Lys Glu Ala Leu
                405                 410                 415

Ala Glu Asn Asn Leu Asn Leu Pro Lys Met Ala Glu Lys Asp Gly Cys
            420                 425                 430

Phe Gln Ser Gly Phe Asn Glu Glu Thr Cys Leu Val Lys Ile Ile Thr
        435                 440                 445

Gly Leu Leu Glu Phe Glu Val Tyr Leu Glu Tyr Leu Gln Asn Arg Phe
    450                 455                 460

Glu Ser Ser Glu Glu Gln Ala Arg Ala Val Gln Met Ser Thr Lys Val
465                 470                 475                 480

Leu Ile Gln Phe Leu Gln Lys Lys Ala Lys Asn Leu Asp Ala Ile Thr
                485                 490                 495

Thr Pro Asp Pro Thr Thr Asn Ala Ser Leu Leu Thr Lys Leu Gln Ala
            500                 505                 510

Gln Asn Gln Trp Leu Gln Asp Met Thr Thr His Leu Ile Leu Arg Ser
        515                 520                 525

Phe Lys Glu Phe Leu Gln Ser Ser Leu Arg Ala Leu Arg Gln Met
    530                 535                 540

<210>  7
<211>  553
<212>  PRT
<213>  Artificial

<220>
<223>  sIL-6R/IL-6 with linker EFGAGLVLGGQFM, Chebath et al. 1997

<400>  7

Met Leu Ala Val Gly Cys Ala Leu Leu Ala Ala Leu Leu Ala Ala Pro
1               5                   10                  15

Gly Ala Ala Leu Ala Pro Arg Arg Cys Pro Ala Gln Glu Val Ala Arg
            20                  25                  30

Gly Val Leu Thr Ser Leu Pro Gly Asp Ser Val Thr Leu Thr Cys Pro
        35                  40                  45

Gly Val Glu Pro Glu Asp Asn Ala Thr Val His Trp Val Leu Arg Lys
    50                  55                  60

Pro Ala Ala Gly Ser His Pro Ser Arg Trp Ala Gly Met Gly Arg Arg
65                  70                  75                  80

Leu Leu Leu Arg Ser Val Gln Leu His Asp Ser Gly Asn Tyr Ser Cys
                85                  90                  95

Tyr Arg Ala Gly Arg Pro Ala Gly Thr Val His Leu Leu Val Asp Val
            100                 105                 110

Pro Pro Glu Glu Pro Gln Leu Ser Cys Phe Arg Lys Ser Pro Leu Ser
        115                 120                 125

Asn Val Val Cys Glu Trp Gly Pro Arg Ser Thr Pro Ser Leu Thr Thr
    130                 135                 140

Lys Ala Val Leu Leu Val Arg Lys Phe Gln Asn Ser Pro Ala Glu Asp
145                 150                 155                 160

Phe Gln Glu Pro Cys Gln Tyr Ser Gln Glu Ser Gln Lys Phe Ser Cys
                165                 170                 175

Gln Leu Ala Val Pro Glu Gly Asp Ser Ser Phe Tyr Ile Val Ser Met
            180                 185                 190

Cys Val Ala Ser Ser Val Gly Ser Lys Phe Ser Lys Thr Gln Thr Phe
        195                 200                 205

Gln Gly Cys Gly Ile Leu Gln Pro Asp Pro Pro Ala Asn Ile Thr Val
    210                 215                 220

Thr Ala Val Ala Arg Asn Pro Arg Trp Leu Ser Val Thr Trp Gln Asp
225                 230                 235                 240

Pro His Ser Trp Asn Ser Ser Phe Tyr Arg Leu Arg Phe Glu Leu Arg
                245                 250                 255

Tyr Arg Ala Glu Arg Ser Lys Thr Phe Thr Thr Trp Met Val Lys Asp
            260                 265                 270

Leu Gln His His Cys Val Ile His Asp Ala Trp Ser Gly Leu Arg His
        275                 280                 285

Val Val Gln Leu Arg Ala Gln Glu Glu Phe Gly Gln Gly Glu Trp Ser
    290                 295                 300

Glu Trp Ser Pro Glu Ala Met Gly Thr Pro Trp Thr Glu Ser Arg Ser
305                 310                 315                 320

Pro Pro Ala Glu Asn Glu Val Ser Thr Pro Met Gln Ala Leu Thr Thr
                325                 330                 335

Asn Lys Asp Asp Asp Asn Ile Leu Phe Arg Asp Ser Ala Asn Ala Thr
            340                 345                 350

Ser Leu Pro Val Glu Phe Gly Ala Gly Leu Val Leu Gly Gly Gln Phe
        355                 360                 365

Met Pro Val Pro Pro Gly Glu Asp Ser Lys Asp Val Ala Ala Pro His
    370                 375                 380

Arg Gln Pro Leu Thr Ser Ser Glu Arg Ile Asp Lys Gln Ile Arg Tyr
385                 390                 395                 400

Ile Leu Asp Gly Ile Ser Ala Leu Arg Lys Glu Thr Cys Asn Lys Ser
                405                 410                 415

Asn Met Cys Glu Ser Ser Lys Glu Ala Leu Ala Glu Asn Asn Leu Asn
            420                 425                 430

Leu Pro Lys Met Ala Glu Lys Asp Gly Cys Phe Gln Ser Gly Phe Asn
        435                 440                 445

Glu Glu Thr Cys Leu Val Lys Ile Ile Thr Gly Leu Leu Glu Phe Glu
    450                 455                 460

Val Tyr Leu Glu Tyr Leu Gln Asn Arg Phe Glu Ser Ser Glu Glu Gln
465                 470                 475                 480

Ala Arg Ala Val Gln Met Ser Thr Lys Val Leu Ile Gln Phe Leu Gln
                485                 490                 495

Lys Lys Ala Lys Asn Leu Asp Ala Ile Thr Thr Pro Asp Pro Thr Thr
            500                 505                 510

Asn Ala Ser Leu Leu Thr Lys Leu Gln Ala Gln Asn Gln Trp Leu Gln
        515                 520                 525

Asp Met Thr Thr His Leu Ile Leu Arg Ser Phe Lys Glu Phe Leu Gln
    530                 535                 540

Ser Ser Leu Arg Ala Leu Arg Gln Met
545                 550

<210>  8
<211>  540
<212>  PRT
<213>  Artificial

<220>
<223>  sIL-6R/IL-6 without linker, Chebath et al., 1997

<400>  8

Met Leu Ala Val Gly Cys Ala Leu Leu Ala Ala Leu Leu Ala Ala Pro
1               5                   10                  15

Gly Ala Ala Leu Ala Pro Arg Arg Cys Pro Ala Gln Glu Val Ala Arg
            20                  25                  30

Gly Val Leu Thr Ser Leu Pro Gly Asp Ser Val Thr Leu Thr Cys Pro
        35                  40                  45

Gly Val Glu Pro Glu Asp Asn Ala Thr Val His Trp Val Leu Arg Lys
    50                  55                  60

Pro Ala Ala Gly Ser His Pro Ser Arg Trp Ala Gly Met Gly Arg Arg
65                  70                  75                  80

Leu Leu Leu Arg Ser Val Gln Leu His Asp Ser Gly Asn Tyr Ser Cys
                85                  90                  95

Tyr Arg Ala Gly Arg Pro Ala Gly Thr Val His Leu Leu Val Asp Val
        100                 105                 110

Pro Pro Glu Glu Pro Gln Leu Ser Cys Phe Arg Lys Ser Pro Leu Ser
    115                 120                 125

Asn Val Val Cys Glu Trp Gly Pro Arg Ser Thr Pro Ser Leu Thr Thr
    130                 135                 140

Lys Ala Val Leu Leu Val Arg Lys Phe Gln Asn Ser Pro Ala Glu Asp
145                 150                 155                 160

Phe Gln Glu Pro Cys Gln Tyr Ser Gln Glu Ser Gln Lys Phe Ser Cys
                165                 170                 175

Gln Leu Ala Val Pro Glu Gly Asp Ser Ser Phe Tyr Ile Val Ser Met
        180                 185                 190

Cys Val Ala Ser Ser Val Gly Ser Lys Phe Ser Lys Thr Gln Thr Phe
    195                 200                 205

Gln Gly Cys Gly Ile Leu Gln Pro Asp Pro Pro Ala Asn Ile Thr Val
    210                 215                 220

Thr Ala Val Ala Arg Asn Pro Arg Trp Leu Ser Val Thr Trp Gln Asp
225                 230                 235                 240

Pro His Ser Trp Asn Ser Ser Phe Tyr Arg Leu Arg Phe Glu Leu Arg
                245                 250                 255

Tyr Arg Ala Glu Arg Ser Lys Thr Phe Thr Thr Trp Met Val Lys Asp
        260                 265                 270

Leu Gln His His Cys Val Ile His Asp Ala Trp Ser Gly Leu Arg His
    275                 280                 285

Val Val Gln Leu Arg Ala Gln Glu Glu Phe Gly Gln Gly Glu Trp Ser
    290                 295                 300

Glu Trp Ser Pro Glu Ala Met Gly Thr Pro Trp Thr Glu Ser Arg Ser
305                 310                 315                 320

Pro Pro Ala Glu Asn Glu Val Ser Thr Pro Met Gln Ala Leu Thr Thr
                325                 330                 335

Asn Lys Asp Asp Asp Asn Ile Leu Phe Arg Asp Ser Ala Asn Ala Thr
        340                 345                 350

Ser Leu Pro Val Pro Val Pro Pro Gly Glu Asp Ser Lys Asp Val Ala
        355                 360                 365

Ala Pro His Arg Gln Pro Leu Thr Ser Ser Glu Arg Ile Asp Lys Gln
    370                 375                 380

Ile Arg Tyr Ile Leu Asp Gly Ile Ser Ala Leu Arg Lys Glu Thr Cys
385                 390                 395                 400

Asn Lys Ser Asn Met Cys Glu Ser Ser Lys Glu Ala Leu Ala Glu Asn
                405                 410                 415

Asn Leu Asn Leu Pro Lys Met Ala Glu Lys Asp Gly Cys Phe Gln Ser
            420                 425                 430

Gly Phe Asn Glu Glu Thr Cys Leu Val Lys Ile Ile Thr Gly Leu Leu
        435                 440                 445

Glu Phe Glu Val Tyr Leu Glu Tyr Leu Gln Asn Arg Phe Glu Ser Ser
    450                 455                 460

Glu Glu Gln Ala Arg Ala Val Gln Met Ser Thr Lys Val Leu Ile Gln
465                 470                 475                 480

Phe Leu Gln Lys Lys Ala Lys Asn Leu Asp Ala Ile Thr Thr Pro Asp
                485                 490                 495

Pro Thr Thr Asn Ala Ser Leu Leu Thr Lys Leu Gln Ala Gln Asn Gln
            500                 505                 510

Trp Leu Gln Asp Met Thr Thr His Leu Ile Leu Arg Ser Phe Lys Glu
        515                 520                 525

Phe Leu Gln Ser Ser Leu Arg Ala Leu Arg Gln Met
    530                 535                 540

<210> 9
<211> 520
<212> PRT
<213> Artificial

<220>
<223> Fusion of human sIL-6R/IL-6 with 13 aa linker

<400> 9

Met Leu Ala Val Gly Cys Ala Leu Leu Ala Ala Leu Leu Ala Ala Pro
1               5                   10                  15

Gly Ala Ala Leu Ala Pro Arg Arg Cys Pro Ala Gln Glu Val Ala Arg
            20                  25                  30

Gly Val Leu Thr Ser Leu Pro Gly Asp Ser Val Thr Leu Thr Cys Pro
        35                  40                  45

Gly Val Glu Pro Glu Asp Asn Ala Thr Val His Trp Val Leu Arg Lys
    50                  55                  60

Pro Ala Ala Gly Ser His Pro Ser Arg Trp Ala Gly Met Gly Arg Arg
65                  70                  75                  80

Leu Leu Leu Arg Ser Val Gln Leu His Asp Ser Gly Asn Tyr Ser Cys
                85                  90                  95

Tyr Arg Ala Gly Arg Pro Ala Gly Thr Val His Leu Leu Val Asp Val
            100                 105                 110

Pro Pro Glu Glu Pro Gln Leu Ser Cys Phe Arg Lys Ser Pro Leu Ser
        115                 120                 125

Asn Val Val Cys Glu Trp Gly Pro Arg Ser Thr Pro Ser Leu Thr Thr
    130                 135                 140

Lys Ala Val Leu Leu Val Arg Lys Phe Gln Asn Ser Pro Ala Glu Asp
145                 150                 155                 160

Phe Gln Glu Pro Cys Gln Tyr Ser Gln Glu Ser Gln Lys Phe Ser Cys
                165                 170                 175

Gln Leu Ala Val Pro Glu Gly Asp Ser Ser Phe Tyr Ile Val Ser Met
            180                 185                 190

Cys Val Ala Ser Ser Val Gly Ser Lys Phe Ser Lys Thr Gln Thr Phe
        195                 200                 205

Gln Gly Cys Gly Ile Leu Gln Pro Asp Pro Pro Ala Asn Ile Thr Val
    210                 215                 220

Thr Ala Val Ala Arg Asn Pro Arg Trp Leu Ser Val Thr Trp Gln Asp
225                 230                 235                 240

Pro His Ser Trp Asn Ser Ser Phe Tyr Arg Leu Arg Phe Glu Leu Arg
                245                 250                 255

Tyr Arg Ala Glu Arg Ser Lys Thr Phe Thr Thr Trp Met Val Lys Asp
            260                 265                 270

Leu Gln His His Cys Val Ile His Asp Ala Trp Ser Gly Leu Arg His
        275                 280                 285

Val Val Gln Leu Arg Ala Gln Glu Glu Phe Gly Gln Gly Glu Trp Ser
    290                 295                 300

Glu Trp Ser Pro Glu Ala Met Gly Thr Pro Trp Thr Glu Ser Arg Ser
305                 310                 315                 320

Pro Pro Ala Arg Gly Gly Gly Gly Ser Gly Gly Gly Gly Ser Val Glu
                325                 330                 335

Pro Val Pro Pro Gly Glu Asp Ser Lys Asp Val Ala Ala Pro His Arg
            340                 345                 350

Gln Pro Leu Thr Ser Ser Glu Arg Ile Asp Lys Gln Ile Arg Tyr Ile
        355                 360                 365

Leu Asp Gly Ile Ser Ala Leu Arg Lys Glu Thr Cys Asn Lys Ser Asn
    370                 375                 380

Met Cys Glu Ser Ser Lys Glu Ala Leu Ala Glu Asn Asn Leu Asn Leu
385                 390                 395                 400

Pro Lys Met Ala Glu Lys Asp Gly Cys Phe Gln Ser Gly Phe Asn Glu
                405                 410                 415

Glu Thr Cys Leu Val Lys Ile Ile Thr Gly Leu Leu Glu Phe Glu Val
            420                 425                 430

Tyr Leu Glu Tyr Leu Gln Asn Arg Phe Glu Ser Ser Glu Glu Gln Ala
        435                 440                 445

Arg Ala Val Gln Met Ser Thr Lys Val Leu Ile Gln Phe Leu Gln Lys
    450                 455                 460

Lys Ala Lys Asn Leu Asp Ala Ile Thr Thr Pro Asp Pro Thr Thr Asn
465                 470                 475                 480

Ala Ser Leu Leu Thr Lys Leu Gln Ala Gln Asn Gln Trp Leu Gln Asp
                485                 490                 495

Met Thr Thr His Leu Ile Leu Arg Ser Phe Lys Glu Phe Leu Gln Ser
            500                 505                 510

Ser Leu Arg Ala Leu Arg Gln Met

515                 520

<210>  10
<211>  525
<212>  PRT
<213>  Artificial

<220>
<223>  Fusion of human sIL-6R/IL-6 with 18 aa linker

<400>  10

Met Leu Ala Val Gly Cys Ala Leu Leu Ala Ala Leu Leu Ala Ala Pro
1               5                   10                  15

Gly Ala Ala Leu Ala Pro Arg Arg Cys Pro Ala Gln Glu Val Ala Arg
            20                  25                  30

Gly Val Leu Thr Ser Leu Pro Gly Asp Ser Val Thr Leu Thr Cys Pro
        35                  40                  45

Gly Val Glu Pro Glu Asp Asn Ala Thr Val His Trp Val Leu Arg Lys
    50                  55                  60

Pro Ala Ala Gly Ser His Pro Ser Arg Trp Ala Gly Met Gly Arg Arg
65                  70                  75                  80

Leu Leu Leu Arg Ser Val Gln Leu His Asp Ser Gly Asn Tyr Ser Cys
                85                  90                  95

Tyr Arg Ala Gly Arg Pro Ala Gly Thr Val His Leu Leu Val Asp Val
            100                 105                 110

Pro Pro Glu Glu Pro Gln Leu Ser Cys Phe Arg Lys Ser Pro Leu Ser
        115                 120                 125

Asn Val Val Cys Glu Trp Gly Pro Arg Ser Thr Pro Ser Leu Thr Thr
    130                 135                 140

Lys Ala Val Leu Leu Val Arg Lys Phe Gln Asn Ser Pro Ala Glu Asp
145                 150                 155                 160

Phe Gln Glu Pro Cys Gln Tyr Ser Gln Glu Ser Gln Lys Phe Ser Cys
                165                 170                 175

Gln Leu Ala Val Pro Glu Gly Asp Ser Ser Phe Tyr Ile Val Ser Met
            180                 185                 190

Cys Val Ala Ser Ser Val Gly Ser Lys Phe Ser Lys Thr Gln Thr Phe
        195                 200                 205

Gln Gly Cys Gly Ile Leu Gln Pro Asp Pro Pro Ala Asn Ile Thr Val
    210                 215                 220

Thr Ala Val Ala Arg Asn Pro Arg Trp Leu Ser Val Thr Trp Gln Asp
225                 230                 235                 240

Pro His Ser Trp Asn Ser Ser Phe Tyr Arg Leu Arg Phe Glu Leu Arg
                245                 250                 255

Tyr Arg Ala Glu Arg Ser Lys Thr Phe Thr Thr Trp Met Val Lys Asp
            260                 265                 270

Leu Gln His His Cys Val Ile His Asp Ala Trp Ser Gly Leu Arg His
        275                 280                 285

Val Val Gln Leu Arg Ala Gln Glu Glu Phe Gly Gln Gly Glu Trp Ser
    290                 295                 300

Glu Trp Ser Pro Glu Ala Met Gly Thr Pro Trp Thr Glu Ser Arg Ser
305                 310                 315                 320

Pro Pro Ala Arg Gly Gly Gly Gly Ser Gly Gly Gly Gly Ser Gly Gly
                325                 330                 335

Gly Gly Ser Val Glu Pro Val Pro Pro Gly Glu Asp Ser Lys Asp Val
            340                 345                 350

Ala Ala Pro His Arg Gln Pro Leu Thr Ser Ser Glu Arg Ile Asp Lys
        355                 360                 365

Gln Ile Arg Tyr Ile Leu Asp Gly Ile Ser Ala Leu Arg Lys Glu Thr
    370                 375                 380

Cys Asn Lys Ser Asn Met Cys Glu Ser Ser Lys Glu Ala Leu Ala Glu
385                 390                 395                 400

Asn Asn Leu Asn Leu Pro Lys Met Ala Glu Lys Asp Gly Cys Phe Gln
                405                 410                 415

Ser Gly Phe Asn Glu Glu Thr Cys Leu Val Lys Ile Ile Thr Gly Leu
            420                 425                 430

Leu Glu Phe Glu Val Tyr Leu Glu Tyr Leu Gln Asn Arg Phe Glu Ser
        435                 440                 445

Ser Glu Glu Gln Ala Arg Ala Val Gln Met Ser Thr Lys Val Leu Ile

450                 455                 460

Gln Phe Leu Gln Lys Lys Ala Lys Asn Leu Asp Ala Ile Thr Thr Pro
465                 470                 475                 480

Asp Pro Thr Thr Asn Ala Ser Leu Leu Thr Lys Leu Gln Ala Gln Asn
                485                 490                 495

Gln Trp Leu Gln Asp Met Thr Thr His Leu Ile Leu Arg Ser Phe Lys
            500                 505                 510

Glu Phe Leu Gln Ser Ser Leu Arg Ala Leu Arg Gln Met
        515                 520                 525

<210>  11
<211>  546
<212>  PRT
<213>  Artificial

<220>
<223>  Hypercytokine H11, WO2005/113591

<400>  11

<210>  12
<211>  13
<212>  PRT
<213>  Artificial

<220>
<223>  Peptide linker

<400>  12

Glu Phe Gly Ala Gly Leu Val Leu Gly Gly Gln Phe Met
1               5                   10

<210>  13
<211>  13
<212>  PRT
<213>  Artificial

<220>

<223>  Peptide linker

<400>  13

Arg Gly Gly Gly Gly Ser Gly Gly Gly Gly Ser Val Glu
1               5                   10

<210>  14
<211>  18
<212>  PRT
<213>  Artificial

<220>
<223>  Peptide linker

<400>  14

Arg Gly Gly Gly Gly Ser Gly Gly Gly Gly Ser Gly Gly Gly Gly Ser
1               5                   10                  15

Val Glu

<210>  15
<211>  3792
<212>  DNA
<213>  Artificial

<220>
<223>  Expression cassette comprising the CMV immediate early promoter
        directing expression of Hyper-IL-6 and Neomycin separated by an
        IRES

<400>  15
gactagttat taatagtaat caattacggg gtcattagtt catagcccat atatggagtt      60

ccgcgttaca taacttacgg taaatggccc gcctggctga ccgcccaacg acccccgccc     120

attgacgtca ataatgacgt atgttcccat agtaacgcca atagggactt tccattgacg     180

tcaatgggtg gagtatttac ggtaaactgc ccacttggca gtacatcaag tgtatcatat     240

gccaagtacg ccccctattg acgtcaatga cggtaaatgg cccgcctggc attatgccca     300

gtacatgacc ttatgggact ttcctacttg gcagtacatc tacgtattag tcatcgctat     360

taccatggtg atgcggtttt ggcagtacat caatgggcgt ggatagcggt ttgactcacg     420

gggatttcca agtctccacc ccattgacgt caatgggagt ttgttttggc accaaaatca     480

acgggacttt ccaaaatgtc gtaacaactc cgccccattg acgcaaatgg gcggtaggcg     540

tgtacggtgg gaggtctata taagcagagc tcgtttagtg aaccgtcaga tcgcctggag     600

acgccatcca cgctgttttg acctccatag aagacaccgg gactggacaa ttcgagctcg     660

gtacccaatt cgagctcgcc cggggatgcg gccgctcgac gcatggagtg gtagccgagg     720

aggaagcatg ctggccgtcg gctgcgcgct gctggctgcc ctgctggccg cgccgggagc     780

ggcgctggcc ccaaggcgct gccctgcgca ggaggtggcg agaggcgtgc tgaccagtct 840

gccaggagac agcgtgactc tgacctgccc gggggtagag ccggaagaca atgccactgt 900

tcactgggtg ctcaggaagc cggctgcagg ctcccacccc agcagatggg ctggcatggg 960

aaggaggctg ctgctgaggt cggtgcagct ccacgactct ggaaactatt catgctaccg 1020

ggccggccgc ccagctggga ctgtgcactt gctggtggat gttccccccg aggagcccca 1080

gctctcctgc ttccggaaga gccccctcag caatgttgtt tgtgagtggg gtcctcggag 1140

caccccatcc ctgacgacaa aggctgtgct cttggtgagg aagtttcaga acagtccggc 1200

cgaagacttc caggagccgt gccagtattc ccaggagtcc cagaagttct cctgccagtt 1260

agcagtcccg gagggagaca gctctttcta catagtgtcc atgtgcgtcg ccagtagtgt 1320

cgggagcaag ttcagcaaaa ctcaaacctt tcagggttgt ggaatcttgc agcctgatcc 1380

gcctgccaac atcacagtca ctgccgtggc cagaaacccc cgctggctca gtgtcacctg 1440

gcaagacccc cactcctgga actcatcttt ctacagacta cggtttgagc tcagatatcg 1500

ggctgaacgg tcaaagacat tcacaacatg gatggtcaag gacctccagc atcactgtgt 1560

catccacgac gcctggagcg gcctgaggca cgtggtgcag cttcgtgccc aggaggagtt 1620

cgggcaaggc gagtggagcg agtggagccc ggaggccatg ggcacgcctt ggacagaatc 1680

caggagtcct ccagctcgag gaggtggagg ttctggaggt ggaggttctg tcgagccagt 1740

acccccagga gaagattcca aagatgtagc cgccccacac agacagccac tcacctcttc 1800

agaacgaatt gacaaacaaa ttcggtacat cctcgacggc atctcagccc tgagaaagga 1860

gacatgtaac aagagtaaca tgtgtgaaag cagcaaagag gcactggcag aaaacaacct 1920

gaaccttcca aagatggctg aaaaagatgg atgcttccaa tctggattca atgaggagac 1980

ttgcctggtg aaaatcatca ctggtctttt ggagtttgag gtatacctag agtacctcca 2040

gaacagattt gagagtagtg aggaacaagc cagagctgtg cagatgagta caaaagtcct 2100

gatccagttc ctgcagaaaa aggcaaagaa tctagatgca ataaccaccc ctgacccaac 2160

cacaaatgcc agcctgctga cgaagctgca ggcacagaac cagtggctgc aggacatgac 2220

aactcatctc attctgcgca gctttaagga gttcctgcag tccagcctga gggctcttcg 2280

gcaaatgtag catgggcacg tcgagggatc ttccatacct accagttctg cgcctgcagg 2340

tcgcggccgc atcctctaga gtcgacccga ccgcggacgc tcgactgcag gaattaattc 2400

cgcccccccc cccccccccc cctaacgtta ctggccgaag ccgcttggaa taaggccggt 2460

gtgcgtttgt ctatatgtta ttttccacca tattgccgtc ttttggcaat gtgagggccc 2520

ggaaacctgg ccctgtcttc ttgacgagca ttcctagggg tctttcccct ctcgccaaag 2580

gaatgcaagg tctgttgaat gtcgtgaagg aagcagttcc tctggaagct tcttgaagac 2640

aaacaacgtc tgtagcgacc ctttgcaggc agcggaaccc cccacctggc gacaggtgcc 2700

tctgcggcca aaagccacgt gtataagata cacctgcaaa ggcggcacaa ccccagtgcc 2760

acgttgtgag ttggatagtt gtggaaagag tcaaatggct ctcctcaagc gtattcaaca 2820

aggggctgaa ggatgcccag aaggtacccc attgtatggg atctgatctg gggcctcggt 2880

gcacatgctt tacatgtgtt tagtcgaggt taaaaaaacg tctaggcccc ccgaaccacg 2940

gggacgtggt tttcctttga aaaacacgat gataagcttg ccacaaccat gggatcggcc 3000

attgaacaag atggattgca cgcaggttct ccggccgctt gggtggagag gctattcggc 3060

tatgactggg cacaacagac aatcggctgc tctgatgccg ccgtgttccg gctgtcagcg 3120

caggggcgcc cggttctttt tgtcaagacc gacctgtccg gtgccctgaa tgaactgcag 3180

gacgaggcag cgcggctatc gtggctggcc acgacgggcg ttccttgcgc agctgtgctc 3240

gacgttgtca ctgaagcggg aagggactgg ctgctattgg gcgaagtgcc ggggcaggat 3300

ctcctgtcat ctcaccttgc tcctgccgag aaagtatcca tcatggctga tgcaatgcgg 3360

cggctgcata cgcttgatcc ggctacctgc ccattcgacc accaagcgaa acatcgcatc 3420

gagcgagcac gtactcggat ggaagccggt cttgtcgatc aggatgatct ggacgaagag 3480

catcaggggc tcgcgccagc cgaactgttc gccaggctca aggcgcgcat gcccgacggc 3540

gaggatctcg tcgtgaccca tggcgatgcc tgcttgccga atatcatggt ggaaaatggc 3600

cgcttttctg gattcatcga ctgtggccgg ctgggtgtgg cggaccgcta tcaggacata 3660

gcgttggcta cccgtgatat tgctgaagag cttggcggcg aatgggctga ccgcttcctc 3720

gtgctttacg gtatcgccgc tcccgattcg cagcgcatcg ccttctatcg ccttcttgac 3780

gagttcttct ga 3792

## Description

### FIELD OF THE INVENTION

The present invention relates to tumour therapy. In particular, the present invention relates to cancer vaccine compositions comprising both allogenic cancer cells modified with hyper-cytokines and stem cells for the prophylaxis and treatment of cancer.

### BACKGROUND OF THE INVENTION

### Cancer Immunotherapy

The concept of cancer immunotherapy is based on the ability of the immune system to recognize cancer cells and to affect their growth and expansion. Beside the fact that tumour cells are genetically distinct from their normal counterparts, and should be recognized and eliminated by the immune system, tumour-associated antigens (TAAs) are often poorly immunogenic due to immunoediting (1). This process allows tumours to evolve during continuous interactions with the host immune system, and eventually escape from immune surveillance. Furthermore, the tumour microenvironment consists of immunosuppressive cells, such as T regulatory cells (T-regs), myeloid-derived suppressor cells (MDSCs), tumour-associated macrophages (TAMs), and cancer associated fibroblasts (CAF) that release immunosuppressive factors including IL-6, IL-10, IDO, TGFβ or VEGF (2, 3). Interaction between cancer and stroma cells creates a network of immunosuppressive pathways, while activation of immune defense is inhibited, e.g. by down-regulation of major histocompatibility complexes and lack of co-stimulatory molecules. Phenotypic analyses show that the tumour contains heterogeneous cell populations displaying different karyotype, growth rate, antigenic profiles, gene expression and immunogenicity. Most solid tumours contain a population of slowly dividing cells expressing stem cell markers, referred to as cancer stem/initiating cells (CSC), which are resistant to all conventional treatment regimens and give rise to metastatic tumours (4).

A key to successful immunotherapy is to overcome the local immunosuppression within tumour microenvironment, and activate mechanisms that lead to tumour eradication. There are two clinical approaches of immunotherapy: active and passive. The passive approach encompasses administration of cytokines, activated effector cells or specific monoclonal antibodies targeting tumour cells. Active immunotherapy involves the stimulation of an immune response towards tumour associated antigens (TAAs), either non-specifically via immunomodulating agents such as antibodies against CTLA-4 (cytotoxic T-lymphocyte associated protein 4) or immune checkpoint inhibitors such as antibodies against PD-1 (programmed cell death protein 1) or PD-L1 (programmed death ligand 1), or specifically employing therapeutic cancer vaccines.

The inventors of the present application studied cancer vaccines designed for mass scale production. They found that the combination of allogeneic cancer cells genetically modified to express a hyper-cytokine, and stem cells in one cancer vaccine composition has a synergistic effect and provides inter alia for (i) increased proliferation specific of CD4⁺ T cells, (ii) increased activation of CD4⁺ T cells, (iii) increased number of memory CD8⁺ and CD 4⁺ T cells, (iv) increased infiltration by dendritic cells and NK cells, (v) decreased infiltration by immunosuppressive monocytes and regulatory T lymphocytes, (vi) increased production of cytokines by T lymphocytes, in particular IL-2, IL-6, and/or IL-10, (vii) increased production of proinflammatory cytokines in the tumour site, in particular IL-12 and IFNγ, (viii) a shift of the immune response towards a Thl type, which is connected with cytotoxic activity, (ix) inhibition of tumour growth, (x) extended progression-free and overall survival. The cancer vaccine compositions of the present invention are therefore be better suited for the treatment of tumours as those known in the prior art.

### SUMMARY OF THE INVENTION

In a first aspect, the present invention relates to a cancer vaccine composition comprising (a) one or more first cancer cells modified to express a first hyper-cytokine, (b) optionally one or more second cancer cells modified to express a second hyper-cytokine, and (c) one or more stem cells, wherein the first and/or second hyper-cytokine is a fusion protein comprising a cytokine receptor and a cytokine.

In a second aspect, the present invention relates to a pharmaceutical composition comprising the cancer vaccine composition according to the first aspect of the invention, additionally comprising pharmaceutically acceptable diluents, carriers, excipients, fillers, binders, lubricants, glidants, disintegrants, adsorbents, and/or preservatives.

In a third aspect the present invention relates to the cancer vaccine composition according to the first aspect of the invention for use in medicine.

In a fourth aspect the present invention relates to the cancer vaccine composition according to the first aspect of the invention for use in a method of treatment of cancer, prevention of recurrence of cancer and/or prevention of cancer.

### DETAILED DESCRIPTION

Before the present invention is described in detail below, it is to be understood that this invention is not limited to the particular methodology, protocols and reagents described herein as these may vary. It is also to be understood that the terminology used herein is for the purpose of describing particular embodiments only, and is not intended to limit the scope of the present invention, which will be limited only by the appended claims. Unless defined otherwise, all technical and scientific terms used herein have the same meanings as commonly understood by one of ordinary skill in the art.

### Tumour vaccines

Tumour vaccines known in the art comprise both non-cell and cell based products. Non-cell based approaches include DNA, peptide, protein vaccines, viral or vector based vaccines, anti-idiotypic antibody vaccines or particle based vaccines. Cell based vaccines include: whole cell vaccines with adjuvants, whole cell genetically modified vaccines (GMTV), dendritic cell (DCs) vaccines pulsed with nucleic acids, peptides, proteins or cell lysates, cancer cell lysates, pulsed DCs with immune stimulators, immune stimulators fused with TAA, cancer cells fused with DCs or lymphocytes B or oncolytic viruses.

The concept of therapeutic cancer vaccines is based on the knowledge that adaptive immunity can be primed and activated to specifically recognize and kill tumour cells. For the last 35 years, several vaccine studies have demonstrated immunological and clinical responses in selected patients, e.g. in renal cell cancer (5). Following the discovery of tumour-associated antigens or DC along with the progress made in molecular biology and biotechnology, which provided recombinant cytokines and gene delivery systems, several strategies of tumour vaccination were proposed: tumour cell-based vaccines consisting of tumour cells admixed with a particular adjuvant (e.g., bacillus Calmette-Guerin, Corynebacterium parvum or IFNs); genetically modified tumour vaccines based on tumour cells expressing genes encoding immune-stimulatory factors; and DCs modified with tumour-derived RNA, loaded with peptides/tumour lysates or fused with tumour cells. Recently, oncolytic virus vaccines were developed and are in number of clinical trials. So far only two therapeutic vaccines obtained the marketing authorisation, Provange (Sipuleucel) based on loaded DC for prostate cancer and T-vec, an oncolytic virus vaccine for melanoma.

### Whole tumour cell vaccines

Cancer vaccines based on irradiated tumour cells deliver a wide spectrum of tumour antigens and may efficiently activate anti-tumour immune responses (6-9) The immunostimulatory potential of irradiated whole-cell vaccines depends on the uptake of tumour antigens by DC and their presentation in regional lymph nodes to activate antigen-specific (as well as bystander) helper and cytotoxic T cells (10). Transduction of vaccine cells with genes encoding immunostimulatory molecules can substantially increase their immunogenicity. In several animal models tumour cells modified with the interleukin-6 (IL-6) gene efficiently activated anti-tumour immune responses (11-13) IL-6 acts on cells through an IL-6 receptor (IL-6R) complex comprising two membrane-bound subunits - α (gp80) and β (gp130). The gp130 subunit is expressed on all cells in the body so far studied (14), while gp80 is expressed on limited number of cells such as hepatocytes, some epithelial cells or some leukocytes. Moreover, soluble variants of gp80 (sIL-6R) and soluble gp130 (sgp130) exist in human plasma. IL-6 may form a dynamic complex with sIL-6R, which directly stimulates gp130-expressing cells (15). There are three activation mechanisms of the IL-6 pathway, *classical, transsignalling* and *cluster signalling. Classical* involves membrane-bound gp80, which attracts gp130 subunits and transduces the signal, while *transsignaling* utilizes a sIL-6R/gp130 complex. IL-6 *transsignalling* regulates mostly pro-inflammatory reactions. The *transsignaling* pathway is more general. Other cells, which do not possess IL-6R are targeted.

### Stem cells (SC)

SC are cells with the ability for self-renewal, as well as the potential to differentiate into multiple cell lines (16). Within the context of the present specification, the term "stem cells" also includes induced pluripotent stem cells (iPSCs) and cancer cell derived stem cells (CDSCs).

### Stem cell classification

SC can be divided by their differentiation and proliferation potential. Totipotent cells develop from the zygote, the most primitive SC, and they can differentiate into any type of cells (17, 18). With the development of the organism blastocyst cells are formed. They possess pluripotent properties and can give rise to all cell types, with the exception of trophoblast cells (17). During embryogenesis, pluripotent cells give rise to multipotent stem cells of individual germ layers, forming endoderm, mesoderm and ectoderm cells. Multipotent cells, often characterized as progenitor cells, are tissue-specific cells, and can give rise to many types of cells characterised by similar properties and origins (19). Oligopotent progenitor cells can only differentiate into several cell types, and bi- or unipotent cells, often referred to as precursor cells, are able to form only two or one specific cell types (20).

SC can also be classified by their origin. Embryonic stem cells (ESC) can be obtained from the blastocyst node a few days after fertilization, prior to implantation of the embryo. The first human ESC cells were isolated in 1998 from embryos generated for *in vitro* fertilization studies and have been characterized as fully pluripotent cells (21). At that time, a significant breakthrough was made in research on human development biology at embryonic and fetal stages, new directions of human pluripotency research, regenerative medicine or new medicinal substances developed, but experiments on human embryonic cells still raise a lot of ethical controversy.

SC are also present in the tissues of an adult organism where they reside in special niches and are called somatic stem cells, e.g. hematopoietic, mesenchymal, epithelial, neuronal SC or skin SC (20, 22). Somatic SC display different degrees of pluripotency - hematopoietic cells have multipotent properties and give rise to the development of all types of blood cells (23). Mesenchymal stem cells can be found in many tissues, e.g. those present in the bone marrow can differentiate into bone cells (osteoblasts and osteoclasts), fat cells (adipocytes), and cartilage cells (chondrocytes) (24). Not all somatic SC display such a wide variety of differentiation potential. Skeletal muscle SC, i.e. muscular satellite cells, are an example of unipotent cells capable of transforming themselves *in vivo* only into muscle cells (25).

### Induced pluripotent stem cells (iPSC)

iPSCs are stem cells obtainable by reprogramming of somatic cells. The first human induced pluripotent stem cells (hiPSC) were obtained in 2007 by reprogramming somatic cells of an adult organism with a retroviral vector, carrying overexpression of four embryonic transcription factors: OCT3/4 (octamer)-binding transcription factor 3/4), SOX2 (sex determining region Y) -box 2), KLF4 (kruppel-like factor 4) and C-MYC (avian myelocytomatosis virus oncogene cellular homolog), in brief, described as OSKM factors (26) (patent details). hiPSC are very similar to hESC cells in terms of morphology, gene expression, epigenetic expression pattern of pluripotency associated genes, antigens presented on the cell surface, proliferation or telomerase activity (27, 28). In addition, hiPSC can differentiate into cells of the three germ layers and form teratomas (26). Development of a method of obtaining iPSC from somatic cells has opened new possibilities for the generation of patient and disease specific hiPSC, enabling their use in disease modelling, toxicology, safety and effectiveness research of new drugs, disease therapies and regenerative medicine. At the same time, the use of iPSC does not raise as much controversy as the use of hESC derived from the human embryo.

The original method of reprogramming somatic cells into iPSC used fibroblasts transduction as described above (26). Of the four OSKM reprogramming factors only OCT3/4 and SOX2 are necessary to initiate reprogramming and generation of iPSC. Their role is based on mutual regulation of expression and activation of pluripotency genes (e.g. Nanog). KLF4 and cMYC are factors that increase efficiency and speed up the reprogramming process (29). After this discovery many attempts have been made to silence or remove transgene sequence from reprogrammed cells, and to increase reprogramming efficiency. Lentiviral vectors became the preferred transgene delivery method, because of their ability to transduce non-dividing cell, what cannot be achieved using retroviral once. However, there are many concerns regarding lentiviral transduction, for example random and stable transgene integration. To overcome this problem, several methods of transgene excision have been developed. One of them is based on the use of naturally occurring transposons that have the ability to move within the genome in a process called transposition. Currently, two methods are used, based on piggyBAC and sleeping beauty transposons, both very efficient in reprogramming somatic cells (30). The transgene removal from the genome is possible by the use of a transposase. Some of vectors carried also loxP sites, which enabled transgene excision by Cre-recombinase overexpression (31). Adenovirus is a non-integrating virus, thus it seems to be a perfect delivery system for reprogramming somatic cells, but reprogramming efficiency of this method is a very serious limitation excluding adenovirus from effective delivery protocols (32).

### Cancer stem cells

Recently, much attention is brought to cancer stem cells as targets for various therapies. Cancer stem cells are defined as a small population of less differentiated cancer cells within cancer that is responsible for tumour self-maintenance, has the ability to self-renew and to give rise to heterogenous cancer cell subsets. Cancer stem cells are also known as tumour initiating cells. They have been first identified and described in hematopoietic malignancies (33, 34). Dick and colleagues characterized rare CD34+CD38- cell subpopulation in human acute myeloid leukemia (AML) cells that propagated colonies in culture and engrafted human leukemia in immune-deficient mice. Since then, cancer stem cells were identified in solid tumours including brain, breast, head and neck, liver, pancreas, colon, bladder, prostate carcinomas and melanoma (35-43).

There is increasing evidence that cancer stem cells are resistant to radiation or conventional chemotherapheutic agents, and contribute to tumour relapse, evasion after standard therapeutic modalities (44-46). Various markers have been postulated to distinguish cancer stem cells from rest of the tumour mass. CD44+CD24- has been used for breast cancer stem cells, CD133+ for glioblastoma and colon carcinoma, head and neck, and CD20+, CD271+ or ABCB5+ for melanoma (43, 47, 48). Recently, ALDH isoenzymes which metabolize genotoxic aldehydes, and provide resistance to alkylating chemotherapheutics, has served as intracellular markers of cancer stem cells (49, 50). High ALDH activity has been found in hematopoietic, neural and prostate stem cells as well as in tumour initiating cells of several cancers (50).

### Fusion proteins of cytokines and cytokine receptors

In order to increase and modify potential bioactivity of some molecular agents, the idea of linkage of two soluble naturally existing components has been postulated. Such fusion proteins have already been described. Hyper-IL-6 is an example of a designer agent, which consists of D2 and D3 domain of IL-6 receptor (IL-6 R) α chain connected to IL-6 via polypeptide linker (51). In the case of IL-6, it was observed that the effective concentration of IL-6 and sIL-6 R, which is needed for the stimulation of cells, which lack membrane IL-6 R is very high (52). The stability of IL-6/sIL-6 R complex was enhanced by linking both components in order to create a fusion protein (Hyper-IL-6) (51). Hyper-IL-6 can directly bind to its signal transducing receptor β subunit (gp130, CD130) and enhance IL-6 activity. Hyper-IL-6 is a fully active fusion protein, which mediates responses at 10 to 1000-fold lower dose compared to the combination of sIL-6/sIL-6 R molecules (51).

### AGI-101H vaccine

AGI-101H is an allogeneic human melanoma vaccine where irradiated cells provide priming of melanoma associated antigens and a designer cytokine, Hyper-IL-6 (H6), serves as molecular adjuvant. Genetic modification of vaccine cells has altered their original phenotype towards melanoma stem cells (MSC) like by activation of gp130-JAK1-STAT3-P-OCT4 pathway. Activation of gp130 was also reported in the conversion of non-stem human breast cancer cells into cancer stem-like cells (53).

The role of the individual allogeneic cell lines is to provide allo-antigenic stimulation of the host immune system via presentation of a mixture of HLA allotypes, along with the delivery of self-antigens including MSC, relevant to immunotherapy of melanoma. The rationale is based on evidence that there are shared melanoma tumour antigens that are presented by DC and are cross-primed in the context of the patient own class I molecules to T cells (54). The cells are irradiated with sterilizing dose before cryo-preservation in order to eliminate possibility of in vivo cell proliferation and tumourigenic potential. This appears to enhance the release of H6, while at the same time limiting the release to a period of a few days during which the administered cells undergo apoptosis proximal to the subcutaneous injection site (55). The drug substance, AGI-101H, is a mixture (1:1 ratio) of two irradiated gene-modified human melanoma lines, referred to as Mich1H6 and Mich2H6. These cell lines were derived from cells acquired from the lymph node metastases of two patients, and have been stably retro-virally transduced with cDNA encoding Hyper-IL-6 (H6). The Mich1 cells are HLA-A01:01 and HLA-A02:03 positive (56); Mich2 cells are HLA-A32:01 positive.

H6 binds directly to gp130, which is present on all so far studied cells, including immune cells, while IL-6R on hepatocytes and some leukocytes. Accordingly, it displays different biological activity than IL-6 and a broader target cells repertoire. H6 is strongly involved in priming by enhancing patient's anti-allogeneic CTL response resulting in release of melanoma antigens from previously irradiated vaccine cells, enhancing presentation of cryptic antigens and activation of maturation of DC, inhibition of T regulatory (T_{reg}) cells (CD4+, CD25+, FoxP3+) formation by down regulation of FoxP3+ by suppression of TGF-β receptor expression on CD4+ lymphocytes (57, 58). H6 activates melanoma antigen specific CTL and NK cells, and increases memory CD8+ and CD4+ lymphocytes. In two studies of the effect of H6 modified cells on the cytokine production of human peripheral blood lymphocytes after allogeneic stimulation, the immune response was polarized towards a Th1 type response. Stimulation of myeloid lineage cells by H6 will also lead to release of GM-CSF, which may promote the expression of MHC - I and II on antigen-presenting cells. That GM-CSF is important in the anti-melanoma effect of H6 has been demonstrated in mice (59). The release of other cytokines from stimulated T-cells, including interferon-gamma and IL-12 may also promote antigen presentation leading to CTL development.

A study of the cytokines released by the Mich1H6 and Mich2H6 cell lines indicated that the modification to include the H6 gene resulted in up-regulation of the expression of a variety of cytokines, including IL-2, IL-6, IL-8, IL-10 and RANTES. Accordingly, the paracrine and autocrine H6 stimulation may result in up-regulation of the immune responses (priming) to both allo-antigens and self-antigens via both direct and indirect mechanisms. This is a critical feature of the AGI-101H mode of action (MoA), since there is no requirement for H6 to enter the systemic circulation to be effective; and prophylactic or therapeutic responses can be achieved at sub-toxic doses. Antigen-specific T-cells may then leave the local lymph nodes and migrate via the blood stream to encounter circulating or depository cancer cells in various tissues and distal metastatic tumour cells. However, cancer cells, including melanoma, forming a tumour use various mechanisms including activation of immune check-points to eliminate entering specific CTLs. Blockade of check-points synapse (for ex. by anti-PD-1 or anti PD-1L antibodies) inhibits elimination of CTLs what may lead with time to their exhaustion and apoptosis finally the progression of cancer. Accordingly, the maintenance of effective specific immune response requires continuous boosting with increased intensity in the case of progression. Continuous vaccine administrations (boosting) activate resting or memory T-cells, to increase the total number of antigen-specific T-cells and maintain anti-tumour cytotoxic activity, since only recently activated T-cells can extravasate and infiltrate the tumour tissue.

The unique feature of AGI-101H vaccine is the expression and high activity of ALDHA1 that may account for direct induction of immune response targeting cancer CSC. The inventors have recently demonstrated the specific T-cell immune response elicited against MAA and CSC (ALDH1 antigen) in AGI-101H vaccinated patients (60). Moreover, anti-ALDH1 antibodies were generated and found in serum of immunized patients.

Moreover, AGI-101H induces the expression of BCL-6 transcriptional repressor in peripheral T lymphocytes that significantly negatively correlates with the level of BCL6 target genes. BCL-6 strongly enforces the progenitor-like fate of T cells and represses the exhaustion of cancer-experienced T cells, which are further responsible for therapeutic effects of AGI-101H administration (61). Therefore, cancer antigen-experienced T cells may fulfill their role protecting immunized patients against melanoma relapse for many years (even decades).

### AGI-101H + iPS vaccine

The inventors of the present application studied tumour vaccines designed for mass scale production. The inventors found in an animal model that a cancer vaccine composition comprising a tumour cell line genetically modified to express a hyper-cytokine and stem cells (in particular allogeneic iPSCs or allogeneic tumour cells derived from spheres) are much more effective in the prophylaxis and treatment of cancer, such as melanoma, than a vaccine that comprises genetically modified tumour cells but no stem cells. The inventors found that vaccination using the cancer vaccine composition according to the invention generated cytotoxic T lymphocytes specific for melanoma stem cells and differentiated melanoma cells, which were moreover, more resistant to exhaustion. The cancer vaccine composition according to the invention is also a suitable drug for the treatment of cancer, prevention of cancer and for preventing recurrence of cancer.

In the following, the elements of the present invention will be described. These elements are listed with specific embodiments, however, it should be understood that they may be combined in any manner and in any number to create additional embodiments. The variously described examples and preferred embodiments should not be construed to limit the present invention to only the explicitly described embodiments. This description should be understood to support and encompass embodiments, which combine the explicitly described embodiments with any number of the disclosed and/or preferred elements. Furthermore, any permutations and combinations of all described elements in this application should be considered disclosed by the description of the present application unless the context indicates otherwise.

Several documents are cited throughout the text of this specification. Each of the documents cited herein (including all patents, patent applications, scientific publications, manufacturer's specifications, instructions, etc.), whether supra or infra, are hereby incorporated by reference in their entirety. Nothing herein is to be construed as an admission that the invention is not entitled to antedate such disclosure by virtue of prior invention.

To practice the present invention, unless otherwise indicated, conventional methods of chemistry, biochemistry, and recombinant DNA techniques are employed which are explained in the literature in the field (62).

Preferably, the terms used herein are defined as described in "A multilingual glossary of biotechnological terms: (IUPAC Recommendations)"(63).

Throughout this specification and the claims which follow, unless the context requires otherwise, the word "comprise", and variations such as "comprises" and "comprising", will be understood to imply the inclusion of a stated integer or step or group of integers or steps but not the exclusion of any other integer or step or group of integer or step. As used in this specification and the appended claims, the singular forms "a", "an", and "the" include plural referents, unless the content clearly dictates otherwise.

The terms "tumour vaccine" and "cancer vaccine" are used interchangeably in the present specification.

The term "stem cell" as used in the present specification refers to cells that have the ability to go through numerous cycles of cell division while maintaining an undifferentiated state (self-renewal) and to differentiate into specialized cell types (potency). The expression of specific markers (stem cell markers) and the capability to both self-renew while maintaining an undifferentiated state and to differentiate into specialized cell types are also referred to as "stem cell characteristics" or "stem cell like properties". The term "stem cell" includes totipotent and pluripotent stem cells, which are able to differentiate into any mature cell type, multipotent stem cells, which are less potent but still able to differentiate into a plurality of different mature cell types and unipotent stem cells, which are able to differentiate into one cell type only. The term "stem cell" as used in the present specification also includes induced pluripotent stem cells (iPSCs) and cancer cell derived stem cells (CDSCs). A preferred example of a CDSC is a cancer cell in which stem cell properties have been induced by culturing the cancer cell in a sphere culture.

The skilled person is aware how to identify stem cells according to the invention by expression of stem cell and pluripotency markers. Useful stem cell and pluripotency markers are: AA4, AA4.1, P-gp (CD243), ABCB5, ABCG2 (CDw338), ALDH, alkaline phosphatase, alpha6-integrin, antithrombin III (AT), asialo GM1, Bcl-2, betal-integrin, c-kit (CD 117), c-Met, C1qR(p), END (CD105), PROM1 (CD133), ALCAM (CD166), ITGB1 (CD29), TNFRSF8 (CD30), PECAM-1 (CD31), Siglec-3 (CD33), CD34, CD44, NCAM (CD56), CD73, CD9, CD90, CDCP1, circulating anticoagulants protein C (PC), CK19, CLV3, cyclic CMP, E-Cadherin, EPCAM, ECMA-7, EDR1, EEC, FGF-4, Flk-2, Flk1(+), Flt3/Flk2, FMS (CD115), FORSE-1, G alpha16, GDF3, GFPM, Gli2, Gli3, glial fibrillary acidic protein, glycoprotein IB, GSTA1, HAS2, Her5, hMYADM, HSA, hsp25, Id2, IL-3Ralpha, Integrins, interleukin-3 receptor alpha chain, KDR Keratin 15, Keratin 19, Kit, L-selectin (CD62L), Lamin A/C, Lewis X antigen, Lgr5, Lrp4, MCM2, MCSP, monosomy 7, MRP4, Msi-1, Musashi, Musashi-1, Mutant BCRP, Nanog, nestin, neurofilament microtubule-associated protein 2, neuron-glial antigen 2 (NG2), notch 1, nrp-1, nucleostemin, Oct-3/4, OST-PTP, P-gp/MDR1, p21, p63, p75, PCLP, PCNA, PECAM, PgP-1, phosphorylated p38, podocalyxin, procalcitonin (PCT), PSCs, PTPRC, RC1 antigen, Rex-1, Sca-1, SCF, sialyl-lactotetra, side population (SP), SOX10, SOX2, SOX9, SP phenotype, SSEA-1, SSEA-3, SSEA-4, Stat3, Stat5, Stella, Stra8, Stro-1, tartrate-resistant acid phosphatase (TRAcP), TdT, telomerase reverse transcriptase, electrophoretic pattern of hemoglobin, Thrombomucin, Thy-1, Tra-1-60, TWIST1, VEGFR-2, vimentin, X-Smoothened, XKrk1 and Zac1. In addition stem cells according to the present invention show no expression or reduced expression of differentiation markers, i.e. genes that are only expressed by certain specialized cell types.

The term "induced pluripotent stem cell (iPSC)" as used in the present specification refers to cells obtained by reprogramming of somatic cells (see above).

The term "cancer cell derived stem cell (CDSC)" as used in the present specification refers to cells obtained by inducing stem cell characteristics in tumour cells. This can be achieved by cultivating tumour cells in a so-called "sphere culture". Most tumour cells form spheres when cultured in non-adherent, low oxygen conditions and in the presence of growth factors. Inside these spheres a hypoxic environment prevails and the cells acquire a stem cell phenotype that manifests in changes of stem cell marker expression. Cells obtained from spheres have acquired aggressiveness and resistance to standard therapeutic approaches such as chemical agents or irradiation while maintaining the landscape of their own differentiated cancer antigens. Accordingly, they provide a broad spectrum of antigens to activate specific immune responses. Moreover, some antigens may be mutated, what further increases the immunogenicity of the cancer vaccines.

The term "hyper-cytokine" as used in the present specification refers to a fusion protein comprising, essentially consisting or consisting of (a) a soluble part of a cytokine receptor, and (b) a cytokine which can bind under physiological conditions to said soluble part of a cytokine receptor and an optional peptide linker positioned between the soluble cytokine receptor and the cytokine. Preferred pairs of a soluble part of a cytokine receptor are IL-6 and sIL-6R, IL-11 and sIL-11R. Within the fusion protein it is preferred that the soluble cytokine receptor is at the N-terminus of the fusion protein and that the cytokine is at the C-terminus. As indicated above the hyper-cytokine optionally may comprise a peptide linker positioned between the cytokine receptor and the cytokine. Said peptide linker may have a length of between 1 to 30 amino acids. Preferably, said peptide linker has a low immunogenicity or is non-immunogenic. More preferably, said peptide linker is non-immunogenic to human beings. The immunogenicity of a peptide linker is influenced by its amino acid sequence. Generally, Gly and Ala elicit a low or no immunogenic response. Thus, it is preferred that at least 50% of the amino acids of the linker are selected from Gly and Ala. Other amino acids that can be present include Ser. Thus, in preferred embodiments at least 80% of the amino acids of the linker are selected from Gly, Ala and Ser. Immunogenicity can be tested by art known methods. Preferred linkers that can be used in the fusion proteins are selected from the group (GGS)ₘ, (GGGS)ₙ and (GGGGS)ₒ, wherein m is an integer between 1 and 10, n is an integer between 1 and 8 and o is an integer between 1 and 5. In some embodiments these linkers are flanked N- terminally by Arg and/or C-terminally by Val, Glu, or Val-Glu.

The term "soluble cytokine receptor" as used in the present specification refers to a soluble fragment of the cytokine receptor, e.g. which lacks most or all of the membrane-spanning part and the cytosolic part and comprises most or all of the extracellular part of the cytokine receptor, as for example sIL-6R. The receptor fragment is soluble, if it is not or essentially not inserted into the membrane of a mammalian cell, preferably a human cell, expressing the receptor fragment.

The term "hyper-cytokine activity" as used in the present specification refers to the activity of the fusion protein. While particularly preferred hyper-cytokines have based on the same molar amount a 10 - to 1000-fold higher activity in the same assays as the cytokine on which they are based or as a mixture of the cytokine and the cytokine receptor. Numerous assays are known to assess the activity of the respective cytokine, which forms the basis for a hyper-cytokine that can be employed in the present invention. If the respective hyper-cytokine has at least 10-fold the activity of the natural occurring cytokine on which it is based (at the same molar amount) or as a mixture of the unfused parts of the cytokine and the soluble part of the cytokine receptor it is considered within the meaning of this invention to exhibit hyper-cytokine activity Suitable assay systems include, e.g. for IL-6 hyper-cytokine the induction of proliferation of BAF-3/cells as described Fischer M. et al. (51). In the context of the present specification, hyper-cytokine IL-6 is also referred to as Hyper-IL-6 or H6.

In a first aspect, the present invention relates to a cancer vaccine composition comprising (a) one or more first cancer cells modified to express a first hyper-cytokine, (b) optionally one or more second cancer cells modified to express a second hyper-cytokine, and (c) one or more stem cells, wherein the first and/or second hyper-cytokine is a fusion protein comprising a cytokine receptor and a cytokine.

The *in vivo* anti tumour effect exerted by compositions comprising (a) or (a) and (b) is significantly enhanced by addition of (c), i.e. by addition of stem cells. Those stem cells may or may not be modified to express a third hyper-cytokine.

In a preferred embodiment of the cancer vaccine composition according to the first aspect of the invention, the composition comprises (a), (b) and (c).

In preferred embodiments, the stem cells are induced pluripotent stem cells (iPSCs) or cancer cell derived stem cells (CDSCs). In preferred embodiments, the stem cells are induced pluripotent stem cells (iPSCs). In preferred embodiments, the stem cells are cancer cell derived stem cells (CDSCs).

In preferred embodiments, the CDSCs are obtained by inducing stem cell like properties in one or more third cancer cells. In particular, the CDSCs are obtained by cultivating tumour cells as spheres as described above.

In preferred embodiments, the first cancer cells (i.e. the cancer cells modified to express a first hyper-cytokine) and the third cancer cells (i.e. the cancer cells from which the CDSCs are obtained), are derived from tissue of two different individuals, preferably from two different humans. It is preferred that the tissue, preferably tumour tissue, is of the same type, e.g. melanoma cells from two different human patients.

In instances where the cancer vaccine composition comprises second cancer cells modified to express a second hyper-cytokine, it is preferred that the second cancer cells and the first and/or third cancer cells, preferably first, second and third cancer cells, are derived from tissue of different individuals, preferably from different humans. It is preferred that the tissue, preferably tumour tissue, is of the same type.

The terms "tissue" or "primary tissue" as used in the present specification refers to both solid tissue like, e.g. skin, liver, brain, kidney, lung, stomach, colon, bladder, or testes, as well as mobile cell populations like, e.g. lymphocytes, or stem cells.

In preferred embodiments, the first, second and/or third cancer cells are independently selected from melanoma cells, renal carcinoma cells, urine bladder cancer cells, prostate cancer cells, colon cancer cells, lung cancer cells, pancreas cancer cells, liver cancer cells, brain cancer cells, head and neck cancer cells, or sarcoma cells.

In preferred embodiments, the first cancer cells and the CDSCs are derived from the same type of cancer. In preferred embodiments, the first and third cancer cells (and if present the second cancer cells) are melanoma cells.

Preferably, the first and third cancer cells (and if present the second cancer cells) are selected from the same type of cancer cell, but are derived either from different tumours within an individual or from two different individuals.

Preferably, the stem cells originate from a different individual that the first (and, if present, the second) cancer cells, preferably an individual having a different HLA type.

In instances where the cancer cells (i.e. the first, second and/or third cancer cells) are derived from primary tissue, preferably a primary tumour, the cells will not all be clonal, but will be composed of one, two, three or more clonal cell populations belonging to a particular cell and/or tumour type. In particular, tumour cells show a high genetic variability upon propagation and, thus, it is common that cells within one established cell line are genetically not entirely identical. Tumour cells originating from one tumour, which have undergone 1, 2, 3, 4, 5, 6, 7, 8, 9, 10 or more cycles of subcultivation *in vitro,* will be less heterogeneous than prior to *in vitro* cultivation, because to *in vitro* cultivation will lead to selection of proliferating cell subtypes and, thus, render the assortment of cells more similar.

In an embodiment, wherein the cancer cells (i.e. the first, second and/or third cancer cells) are derived from primary tissue, preferably the same type of primary tissue, in particular the same tumour tissue, the first, second and/or third cells are considered different if they are derived from two different individuals, preferably from two different humans.

In preferred embodiments, the CDSC is characterized by at least one of: increased Stat3 phosphorylation, increased ALDH1 activity, increased expression of VEGF, CD44 and/or PD-L1 and decreased expression of tyrosinase, MITF and/or MHC in comparison to said third cancer cell. In preferred embodiments, the CDSC is characterized by increased Stat3 phosphorylation, increased ALDH1 activity, increased expression of VEGF, CD44 and PD-L1 and decreased expression of tyrosinase, MITF and/or MHC in comparison to said third cancer cell.

In preferred embodiments, the CDSC is obtained by inducing stem cell like properties in a melanoma cell. In preferred embodiments, the CDSC is obtained by inducing stem cell like properties in a melanoma cell and is characterized by increased Stat3 phosphorylation, increased ALDH1 activity, increased expression of VEGF, CD44 and PD-L1 and decreased expression of tyrosinase, MITF and MHC in comparison to said melanoma cell.

In preferred embodiments, the stem cells are induced pluripotent stem cells (iPSCs).

In preferred embodiments, the iPSCs are obtained by inducing pluripotent stem cell like properties in fibroblasts.

iPS cells can be derived from "healthy" somatic cells (preferably fibroblasts) or differentiated cancer cells by reprogramming cells of an adult organism with a retroviral vector, or lentiviral vector carrying overexpression of four embryonic transcription factors (OCT4, SOX2, KLF4 and C-MYC). Reprograming might also be carried out using non-integrating methods such as, transduction using Sendai virus or RNA transfection. All procedures are carried out in Xenofree conditions. Obtained iPS cells are analyzed for pluripotency markers, correct karyotype and formation of teratoma in animal models. In instances where the iPSCs are modified to express a hyper-cytokine, selected clones are modified with hyper-cytokine (e.g. H6) cDNA and screened for the overexpression of hyper-cytokine protein. Then the highest hyper-cytokine secreting clones are selected and analyzed for genetic stability (by karyotyping) and assessed for the pluripotency status and its stability using teratoma forming assay in mice.

In addition, iPS-hyper-cytokine cells are tested for human pathogens according to GMP requirements and FDA, and EMA recommendations. Fully characterized and propagated cell lines are bio-banked. Next Master and Working Cell Banks are generated, characterized and used for batch manufacturing.

In preferred embodiments, the iPSC is characterized by expression of at least one of the following markers: SSEA-1, Epcam, E-cadherin, NANOG, alkaline phosphatase and/or Oct3/4. In preferred embodiments, the iPSC is characterized by expression of Epcam, E-cadherin, NANOG, alkaline phosphatase and Oct3/4. In preferred embodiments, the iPSC is characterized by expression of SSEA-1, Epcam, E-cadherin, NANOG, alkaline phosphatase and Oct3/4.

In preferred embodiments, the stem cells are modified to express a third hyper-cytokine. The first hyper-cytokine and third hyper-cytokine (and if present the second hyper-cytokine) may be different hyper-cytokines or the same hyper-cytokine.

In preferred embodiments, the first, second and/or third hyper-cytokine is a fusion protein comprising, consisting essentially of or consisting of (a) an IL-6 receptor (IL-6R) part exhibiting at least 90% sequence identity to human soluble IL-6R (sIL-6R), wherein said sequence identity is calculated over the entire length of the polypeptide sequence from P113 to A323 of SEQ ID NO: 1, (b) an IL-6 part exhibiting at least 90% sequence identity to human interleukin-6 (IL-6), wherein said sequence identity is calculated over the entire length of the polypeptide sequence from P29 to M212 of SEQ ID NO: 2, and (c) optionally a peptide linker, wherein the fusion protein has hyper-cytokine activity; preferably at least 10%, at least 20%, at least 30%, at least 40%, at least 50%, at least 60%, at least 70%, at least 80%, at least 90%, at least 100% or more of the activity of the Hyper-IL-6 fusion protein according to SEQ ID NO: 9, when tested in a relevant assay of IL-6 activity, e.g. the induction of proliferation of BAF-3/cells as described in Fischer M. *et al.* (1997).

In preferred embodiments, the first hyper-cytokine is a fusion protein comprising, consisting essentially of or consisting of (a) an IL-6 receptor (IL-6R) part exhibiting at least 90% sequence identity to human soluble IL-6R (sIL-6R), wherein said sequence identity is calculated over the entire length of the polypeptide sequence from P113 to A323 of SEQ ID NO: 1, (b) an IL-6 part exhibiting at least 90% sequence identity to human interleukin-6 (IL-6), wherein said sequence identity is calculated over the entire length of the polypeptide sequence from P29 to M212 of SEQ ID NO: 2, and (c) optionally a peptide linker, wherein the fusion protein has hyper-cytokine activity; preferably at least 10%, at least 20%, at least 30%, at least 40%, at least 50%, at least 60%, at least 70%, at least 80%, at least 90%, at least 100% or more of the activity of the Hyper-IL-6 fusion protein according to SEQ ID NO: 9, when tested in a relevant assay of IL-6 activity, e.g. the induction of proliferation of BAF-3/cells as described in Fischer M. *et al.* (1997).

In preferred embodiments, the first, second and/or third hyper-cytokine is a fusion protein comprising (a) an IL-11 receptor (IL-11R) part exhibiting at least 90% sequence identity to human soluble IL-11 receptor (sIL-11R), wherein said sequence identity is calculated over the entire length of the polypeptide sequence from M1 to Q365 of SEQ ID NO: 3, (b) an IL-11 part exhibiting at least 90% sequence identity to human interleukin-11 (IL-11), wherein said sequence identity is calculated over the entire length of the polypeptide sequence from A19 to L199 of SEQ ID NO: 4, and (c) optionally a peptide linker, wherein the fusion protein has hyper-cytokine activity; preferably at least 10%, at least 20%, at least 30%, at least 40%, at least 50%, at least 60%, at least 70%, at least 80%, at least 90%, at least 100% or more of the activity of the sIL-1 1R-IL-11 fusion protein according to SEQ ID NO: 11, when tested in a relevant assay of IL-11 activity.

In preferred embodiments, the first, second and/or third hyper-cytokine comprises, consists essentially of or consists of (a) a polypeptide having the amino acid sequence according to SEQ ID NO: 5, SEQ ID NO: 6, SEQ ID NO: 7, SEQ ID NO: 8 or SEQ ID NO: 9; or (b) a polypeptide exhibiting at least 90% sequence identity to a polypeptide according to (a) and having hyper-cytokine activity, preferably at least 10%, at least 20%, at least 30%, at least 40%, at least 50%, at least 60%, at least 70%, at least 80%, at least 90%, at least 100% or more of the activity of the unmodified hypercytokine with an amino acid sequence according to SEQ ID NO: 5, SEQ ID NO: 6, SEQ ID NO: 7, SEQ ID NO: 8 or SEQ ID NO: 9. An activity of 100% or more is particularly preferred.

In preferred embodiments, the first hyper-cytokine comprises, consists essentially of or consists of (a) a polypeptide having the amino acid sequence according to SEQ ID NO: 5, SEQ ID NO: 6, SEQ ID NO: 7, SEQ ID NO: 8 or SEQ ID NO: 9; or (b) a polypeptide exhibiting at least 90% sequence identity to a polypeptide according to (a) and having hyper-cytokine activity.

In preferred embodiments, the first and third hyper-cytokine comprises, consists essentially of or consists of (a) a polypeptide having the amino acid sequence according to SEQ ID NO: 5, SEQ ID NO: 6, SEQ ID NO: 7, SEQ ID NO: 8 or SEQ ID NO: 9; or (b) a polypeptide exhibiting at least 90% sequence identity to a polypeptide according to (a) and having hyper-cytokine activity.

In preferred embodiments, the first, second and third hyper-cytokine comprises, consists essentially of or consists of (a) a polypeptide having the amino acid sequence according to SEQ ID NO: 5, SEQ ID NO: 6, SEQ ID NO: 7, SEQ ID NO: 8 or SEQ ID NO: 9; or (b) a polypeptide exhibiting at least 90% sequence identity to a polypeptide according to (a) and having hyper-cytokine activity.

The polypeptide having the amino acid sequence according to SEQ ID NO: 5 consists of the part from P113 to A323 of IL-6R according to SEQ ID NO: 1, a glycine-rich linker sequence of 13 amino acids, and the part from P29 to M212 of IL-6 according to SEQ ID NO: 2 (Fischer et al., 1997). Further sIL-6R and IL-6 fusion proteins having the amino acids sequences according SEQ ID NO: 6, 7 and 8 have been described by Chebath et al. and consist of the region from M1 to V356 of IL-6R according to SEQ ID NO: 1, the region from P29 to M212 of IL-6 according to SEQ ID NO: 2, and different linker sequences. The polypeptide according to SEQ ID NO: 6 comprises a 3 amino acid linker sequence (EFM), the polypeptide according to SEQ ID NO: 7 comprises a 13 amino acid linker sequence (EFGAGLVLGGQFM; SEQ ID NO: 12), and the polypeptide according to SEQ ID NO: 8 contains no linker sequence. Additional, fusion proteins have been described in WO 97/32891 and comprise amino acids M1 to A323 of sIL-6R according to SEQ ID NO: 1 and amino acids and P29 to M212 of IL-6 according to SEQ ID NO: 2 linked by either a 13 amino acid linker sequence (RGGGGSGGGGSVE, SEQ ID NO: 13) according to SEQ ID NO: 9 or a 18 amino acid linker sequence (RGGGGSGGGGSGGGGSVE; SEQ ID NO: 14) according to SEQ ID NO: 10. The fusion protein having the amino acid sequence according to SEQ ID NO: 11 comprises the region from M1 to Q365 of sIL-11R and the region from A19 to L199 of IL-11. The sIL-6R IL-6 fusion according to SEQ ID NO: 9 is a particularly preferred embodiment and is referred herein as "Hyper-IL-6" or "H6". A preferred expression cassette comprising Hyper-IL-6 and the Neo selectable marker both under the control of the CMV immediate early promoter is provided as SEQ ID NO: 15. This cassette may be comprised in a variety of vectors, preferably viral vectors like retroviral vectors.

In preferred embodiments, the first and/or second cancer cell modified to express a first or second hyper-cytokine is a melanoma cell, in particular Mich1-H6, deposited under accession number DSM ACC2839 with DSMZ, or Mich2-H6, deposited under accession number DSM ACC2840 with DSMZ. In one embodiment, the first cancer cell modified to express a first hyper-cytokine is Mich1-H6 and the second cancer cell modified to express a second hyper-cytokine is Mich2-H6. Mich1-H6 and Mich2-H6 were derived by transduction of melanoma cells with a retrovirus comprising the expression cassette according to SEQ ID NO: 15 and expressing Hyper-IL-6 according to SEQ ID NO: 9 under the control of the CMV promoter.

While it is possible that the cells comprised in the cancer vaccine composition are autologous or allogenic, it is preferred that they are allogenic. In preferred embodiments, the first cancer cells, second cancer cells, and/or stem cells are allogenic. In particular, it is preferred that the first cancer cells and the stem cells (and if present the second cancer cells) are allogenic.

The term "allogenic" as used in the present specification characterizes the relation between the cells and a patient receiving the cells. Cells from a particular individual will be allogenic to any other patient, while they will be autologous to that particular individual. Allogenicity is a prerequisite for industrial large-scale production of any cell based vaccine, since otherwise each cellular vaccine would have to be produced individually from cells isolated and cultured from the respective patient to be treated. Allogenic cells provide additional advantages, which include that allogenic cells tend to induce a stronger immune response in a patient than autologous cells.

It is preferred that the proliferation of the cells comprised in the cancer vaccine composition is inhibited in order to increase safety of the cancer vaccine composition. In preferred embodiments, the proliferation of the first cancer cells, second cancer cells, and/or stem cells is inhibited. In preferred embodiments, the proliferation of the first cancer cells, the stem cells (and if present the second cancer cells) is inhibited. In preferred embodiments, the proliferation is inhibited by radioactive irradiation or chemical cross-linking.

In preferred embodiments, the first cancer cells, second cancer cells, and/or stem cells is further modified to express an antigen, a cytokine, anti-TGF, EPO, interferon, in particular INF-a, LIF, OSM, CNTF, CT-1 or an additional hyper-cytokine different from the first, second or third hyper-cytokine expressed by the respective cell. Preferably, the antigen is a tumour antigen. Preferably, the cytokine is GM-CSF, IL-2, IL-6, IL-7, IL-11, IL-15 or IL-21. In preferred embodiments, the first cancer cells and/or stem cells are further modified to express GM-CSF.

The term "modified to express" as used in the present specification indicates that a gene, e.g. a gene encoding a hyper-cytokine, in particular H6, has been stably transduced into the cell in a form that allows stable and long-lasting expression of the gene and, subsequently, production of the respective transgenic protein. Preferably, the gene to be expressed is inserted into an expression vector for use in mammalian cells, which ordinarily include an origin of replication (not always necessary, see below), a promoter located in front of the gene to be expressed, optionally an enhancer in trans, along with any necessary ribosome binding sites, RNA splice sites, polyadenylation site, and transcriptional terminator sequences. Such an expression vector may then be used to modify the cell to express the gene, e.g. a gene encoding a hyper-cytokine, in particular H6.

As indicated above rather than using expression vectors that contain viral origins of replication, cells can be transformed with vectors controlled by appropriate expression control elements (e.g., promoter, enhancer, sequences, transcription terminators, polyadenylation sites, etc.), and a selectable marker. Following the introduction of foreign DNA, engineered cells may be allowed to grow for 1-2 days in an enriched medium, and are then switched to a selective medium. The selectable marker in the recombinant plasmid confers resistance to the selection and allows cells to stably integrate the plasmid into their chromosomes and grow to form foci, which in turn can be cloned and expanded into cell lines.

In a preferred embodiment, the expression vector used to transform, transfect or infect the cell to be modified comprises the gene encoding the selectable marker as one transcript with the gene to be expressed, e.g. the hyper-cytokine gene. To ascertain the individual expression of the selectable marker and the gene to be expressed, e.g. the hyper-cytokine gene, an internal ribosome entry site (IRES) is placed between the two coding sequences.

The cells to be included in the composition of the present invention are preferably propagated separately. Preferably they are propagated *in vitro* in one of two modes: as non-anchorage dependent cells growing in suspension throughout the bulk of the culture or as anchorage-dependent cells requiring attachment to a solid substrate for their propagation (i.e., a monolayer type of cell growth). The appropriate growth conditions are determined by the cell type and can be determined by the skilled person using routine experimentation.

In a further preferred embodiment of the composition of the first or second aspect, the hyper-cytokine comprises, consists essentially of or consists of:
(a) a polypeptide having the amino acid sequence according to SEQ ID NO: 5 to 11; or
(b) a polypeptide exhibiting at least 90% sequence identity to a polypeptide according to (a) and having hyper-cytokine activity.

The human leukocyte antigen system (HLA system) is a gene complex encoding the major histocompatibility complex (MHC) proteins in humans. The HLA gene complex resides on chromosome 6, and encodes cell-surface antigen-presenting proteins. The major HLA antigens are essential elements in immune function.

Different MHC classes have different functions:
(a) class I antigens (A, B & C) present peptides from inside the cell (including viral peptides if present) to T-lymphocytes, and
(b) class II antigens (DR, DP, & DQ) present phagocytosed antigens from outside of the cell to T-lymphocytes.

Aside from the genes encoding the 6 major antigens, there is a large number of other genes located on the HLA complex, many of them involved in immune function. Diversity of HLA in human population is one aspect of disease defense, and, as a result, the chance of two unrelated individuals having identical HLA molecules on all loci is very low. The proteins encoded by HLAs are the proteins on the outer part of body cells that are (effectively) unique to that person. The immune system uses the HLAs to differentiate self-cells and non-self cells. Any cell displaying that individuals' HLA type belongs to that individual (and therefore is not an invader). Donor organs transplanted into recipients elicit antibodies against the donor's tissues and turning the donor's HLA receptors into antigens of the recipient's immune system, hence the name "human leukocyte antigens". The types of receptors could be classified based on the antibodies that they induced. These antibodies, particularly to donors who were homozygotes of a particular class II haplotype can be used to identify different receptor types and isoforms. There are two parallel systems of nomenclature that are used to classify HLA. The, first, and oldest system is based on serological (antibody based) recognition. In this system antigens are eventually assigned letters and numbers (e.g. HLA-B27 or, shortened, B27). A parallel system has been developed that allowed more refined definition of alleles, in this system a "HLA" is used in conjunction with a letter * and four or more digit number (e.g. HLA-B*0801, A*68011, A*240201N N=Null) to designate a specific allele at a given HLA locus. HLA loci can be further classified into MHC class I and MHC class II (or rarely, D locus). This classification is based on sequence information from the respective HLA loci. Accordingly, the skilled person is well aware how to determine, whether two groups of cells have the same or a different HLA type.

The term "tumour antigen" comprises all substances, which elicit an immune response against a tumour. Particular suitable substances are proteins or protein fragments which are enriched in a tumour cell in comparison to a healthy cell. These substances are preferably present within and/or are accessible on the outside of the tumour cell. If the tumour antigen is only present within a tumour cell, it will still be accessible for the immune system, since the antigen or fragments thereof will be presented by the MHC system at the surface of the cell. In a preferred aspect the tumour antigen is almost exclusively or exclusively present on and/or in the tumour cell and not in a healthy cell of the same cell type.

When the composition according to the first, the second, the third and forth aspect of the present invention is administered to a patient it is administered to elicit an immune response both against the first cancer cells (a) and/or the stem cells (c) and against any tumour cells, which share epitopes and/or tumour antigens with the first cancer cells (a) and/or the stem cells. It is, thus, expected that the cells of the compositions will only survive for a limited time within the recipient of the compositions of the present invention and are then cleared from the organism of the recipient by the immune system of the recipient. Nevertheless, it is preferred for safety reasons that proliferation of the first cancer cells (a), second cancer cells (b), and stem cells (c) is inhibited, in particular by radioactive irradiation or chemical cross-linking, prior to the administration of these cells to a patient. The term "inhibition" comprises both the slowing down of the proliferation rate and the complete cessation of proliferation. The skilled person is aware of a larger number of chemical and physical methods, which affect the growth rate of cells, these include without limitation radiation, e.g. γ-irradiation or cross-linking, e.g. psoralen or aldehyde. The level of inhibition, however, should preferably be such, that transcription and translation of the transgenes introduced into the first and second cells is not completely shut down, i.e. the transgenes should be expressed at a level of at least 5%, preferably at least 10%, at least 20%, at least 30%, at least 40%, at least 50%, at least 60%, at least 70%, at least 80%, at least 90%, at least 100% or more of the first and/or second cells prior to inhibition. Preferably, the cells are capable to continue to go through 1 to 5, i.e. 1, 2, 3, 4, or 5, replication cycles after their proliferation is completely inhibited.

As it is intended that the compositions of the present invention elicit an immune response the composition requires adjuvants.

In a second aspect the present invention relates to a pharmaceutical composition comprising the cancer vaccine composition according to the first aspect of the invention, additionally comprising pharmaceutically acceptable diluents, carriers, excipients, fillers, binders, lubricants, glidants, disintegrants, adsorbents, and/or preservatives. Preferably the pharmaceutical composition is formulated for parenteral use, preferably in the form of a sterile aqueous solution, which may contain other substances, for example, enough salts or glucose to make the solution isotonic with blood. The aqueous solutions should be suitably buffered (preferably to a pH of from 3 to 9), if necessary. A particularly preferred aqueous solution is phosphate buffered saline (PBS).

Preferably a unit dose of a composition or pharmaceutical composition of the present invention comprises between at least 1 x 10⁶ and 1 x 10⁸ cells of the first cancer cells (allogeneic tumour cells), preferably at least 1 x 10⁷, 2 x 10⁷, 3 x 10⁷, 4 x 10⁷, 5 x 10⁷, 6 x 10⁷, 7 x 10⁷, 8 x 10⁷, 9 x 10⁷, and 1 x 10⁸. Additionally, the unit dose of a composition or pharmaceutical composition of the present invention comprises between at least 1 x 10⁶ and 1 x 10⁸ cells of the second cancer cells (allogeneic tumour cells), preferably at least 1 x 10⁷, 2 x 10⁷,3 x 10⁷, 4 x 10⁷, 5 x 10⁷, 6 x 10⁷, 7 x 10⁷, 8 x 10⁷, 9 x 10⁷, and 1 x 10⁸. Moreover, the unit dose of a composition or pharmaceutical composition of the present invention comprises between at least 1 x 10⁶ and 1 x 10⁸ cells of stem cells (iPSCs or sphere-stem cells), preferably at least 1 x 10⁷, 2 x 10⁷, 3 x 10⁷, 4 x 10⁷, 5 x 10⁷, 6 x 10⁷, 7 x 10⁷, 8 x 10⁷, 9 x 10⁷, and 1 x 10⁸. A particular preferred unit dose of a composition or pharmaceutical composition of the present invention comprises between 1 x 10⁷ to 1 x 10⁸ second cancer cells, preferably 2 x 1 x 10⁷. Preferably, the composition or pharmaceutical composition of the present invention comprises about the same number of first cancer cells and second cancer cells for a total of 2 x 10⁵ to 2 x 10⁸ cells per unit dose and double amount of (1:2 ratio) of stem cells (iPSCs or sphere stem cells). A particular preferred unit dose of a composition or pharmaceutical composition of the present invention comprises between 1 x 10⁷ to 2 x 10⁸ of each of first cancer cells, second cancer cells, and stem cells, preferably 4 x 10⁷. The total volume of the unit dose is preferably between 0.5 to 10 ml, preferably, 1 to 5 ml, e.g. 1, 2, 3, 4, or 5 ml.

In a third aspect the present invention relates to the cancer vaccine composition according to the first aspect of the invention for use in medicine.

In a fourth aspect the present invention relates to the compositions of the present invention or the pharmaceutical composition of the present invention for the treatment of cancer, the prevention of cancer, and/or the prevention of recurrence of cancer. Preferred cancers treatable or preventable with a composition according to the present invention are selected from the group consisting of cancer of the gastrointestinal or colorectal tract, liver, pancreas, kidney, bladder, prostate, endometrium, head and neck cancer, ovary, testes, prostate, skin, eye, melanoma, dysplastic oral mucosa, invasive oral cancer, small cell and non-small cell lung cancer, hormone-dependent breast cancer, hormone independent breast cancer, transitional and squamous cell cancer, neurological malignancy, including neuroblastoma, glioma, astrocytoma, endocrinological tumour, hematologic neoplasia including leukemia, lymphoma, and other myeloproliferative and lymphoproliferative diseases. Particularly preferred is the treatment or prevention of melanoma, renal cell carcinoma, prostate cancer, colon cancer, lung cancer, pancreas cancer, liver cancer, brain cancer or head and neck cancer. More preferred is the treatment or prevention of melanoma, renal cell cancer, pancreas cancer, urine bladder cancer or lung cancer. Even more preferred is the treatment or prevention of melanoma, pancreas cancer and renal cell cancer.

In a further aspect the present invention relates to the use of the compositions of the present invention for the preparation of a pharmaceutical composition for the treatment of cancer, the prevention of cancer, and/or the prevention of recurrence of cancer.

The compositions of the invention can be used in the treatment and/or prevention of a wide variety of different cancers, however, preferred cancers treatable or preventable according to the present invention are selected from the group consisting of cancer of the gastrointestinal or colorectal tract, liver, pancreas, kidney, bladder, prostate, head and neck cancer, ovary, testes, prostate, skin, melanoma, small cell and non-small cell lung cancer, hormone-dependent breast cancer, hormone independent breast cancer, neurological malignancy, including neuroblastoma, glioma, soft tissue sarcoma, endocrinological tumour, Particularly preferred is the treatment or prevention of melanoma, renal cell carcinoma, prostate cancer, colon cancer, lung cancer, pancreas cancer, liver cancer, brain cancer, head or neck cancer. Even more preferred is the treatment or prevention of melanoma, renal cell cancer, pancreas cancer, urine bladder cancer or lung cancer. Even more preferred is the treatment or prevention of melanoma, pancreas cancer and renal cell cancer. Even more preferred is the treatment or prevention of melanoma.

In preferred embodiments, the composition is administered as a vaccine.

In particular in the context of the treatment and/or prevention of cancer it is envisionable that patients are immunized with a "cancer vaccine composition" prior to the development of any symptoms of a disease, i.e. receive a protective immunization, or after they have developed symptoms of the disease, i.e. receive a therapeutic vaccination. It is further envisioned that the cancer vaccine can be administered to patients after symptoms of the disease have disappeared in said patients in order to prevent recurrence of the disease. In particular, the cancer vaccine composition can be administered to the patients after surgical excision of the tumour to prevent cancer recurrence. The compositions and pharmaceutical compositions of the present invention can be used as such cancer vaccines described in this paragraph.

In preferred embodiments, the patient to be treated has a partially or completely different HLA type than the first cancer cells, second cancer cells, and/or stem cells, in particular a partially or completely different HLA type than the first cancer cells and the stem cells.

In preferred embodiments, the first cancer cells, second cancer cells, and/or stem cells are obtained from the same type of tumour as the one that is to be treated in the patient receiving the cancer vaccine composition.

It is particularly preferred in this context when the first cancer cells and/or the second cancer cells are from the same type of tissue, preferably tumour tissue, but have a partially or completely different HLA type.

The expression of at least one further cytokine, in particular GM-CSF, by the first cancer cells and/or the second cancer cells modified to express a hyper-cytokine, preferably Hyper-IL-6, can provide in the context of certain tumours, in particular melanoma and renal cancer an even stronger *in vivo* anti-tumour response than cells expressing only the hyper-cytokine. Therefore, in a preferred use the first cancer cells and/or the second cancer cells modified to express a hyper-cytokine are further modified to express at least one further cytokine and are used for the manufacturing of medical product to prevent or treat a proliferative disease.

### BRIEF DESCRIPTION OF THE DRAWINGS

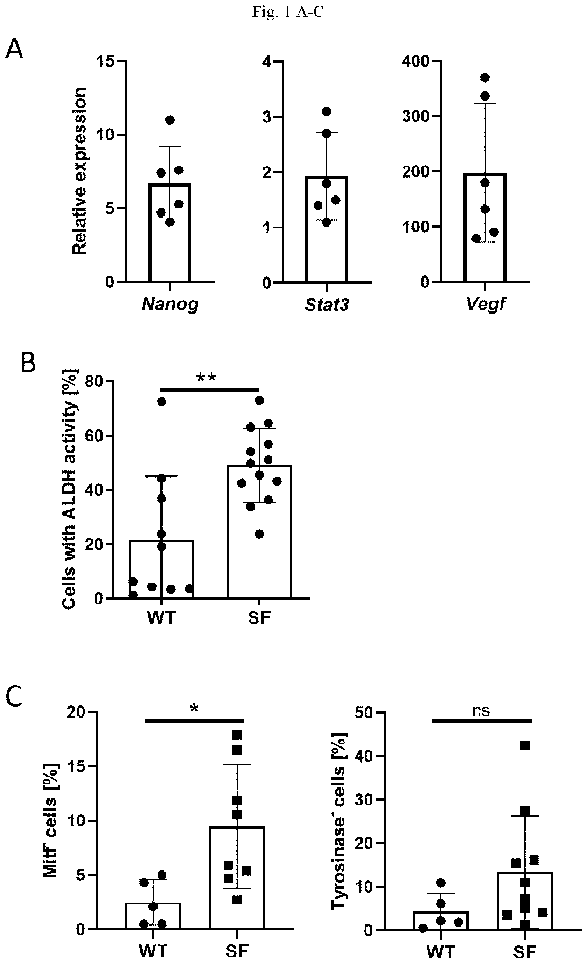

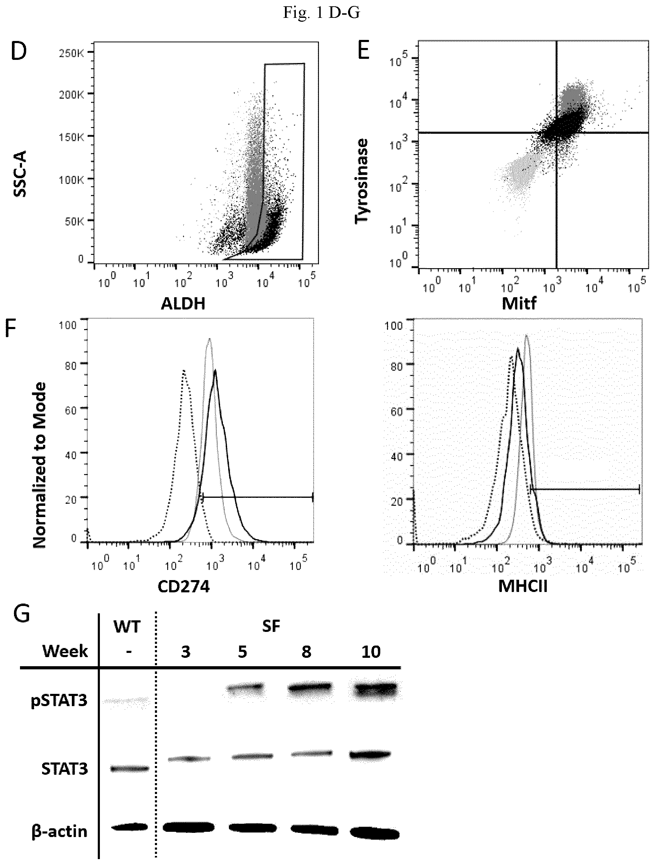

- Fig. 1.: shows that melanoma cells cultured as melanospheres are enriched in stem cell characteristics. Quantitative PCR of *NANOG, MITF, STAT3 and VEGF* in melanospheres (SF) during weeks 5-10 of culture (passage 5-10). Results are normalized to *GAPDH* and presented as fold change relative to adherent culture of B16F10 (WT). (A) Cytometric assay of the number of cells with highly active ALDH in SF and WT, passages 6-10 from three culture cycle; p<0.01. (B) Percentage of MITF- and tyrosinase - negative cells in SF and WT measured by flow cytometry p<0.05. (C) Representative ALDH activity graph comparing SF (black) and WT (grey). Gate drawn according to WT cells with inactivated ALDH. (D) Representative Mitf and tyrosinase expression graph comparing SF (black), WT (grey) and isotype control (light grey). (E) Expression of CD274 and MHCII in SF (black) and WT (grey) compared to isotype control (dotted). (F) Representative Western blot of phosphorylated and total STAT3 protein with β-actin as a housekeeping gene in WT and SF at passage 3, 5, 8, 10

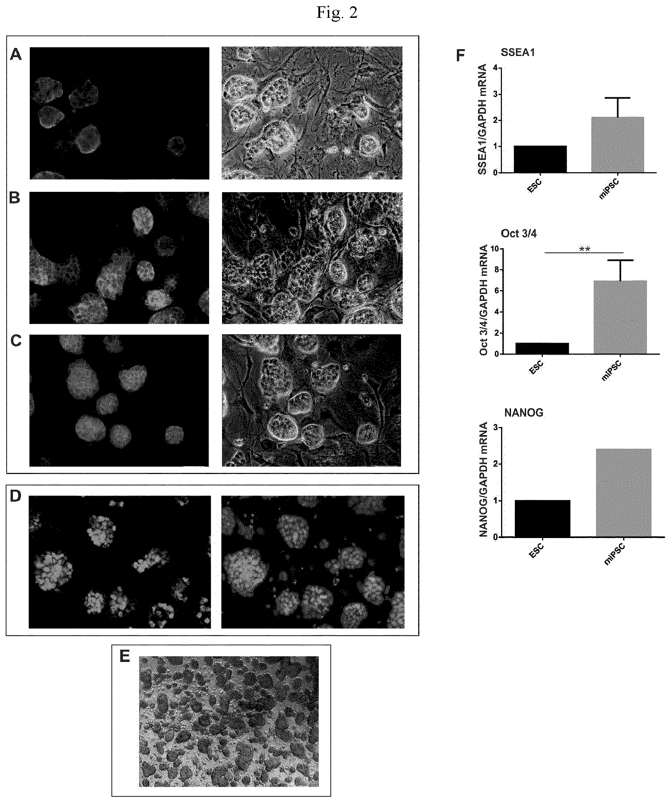

- Fig. 2: shows that iPS cells express pluripotency markers: SSEA-1 (A), Epcam (B), E-cadherin (C), (fluorescence microscope image (first column), phase-contrast microscope image (second column)); Nanog (D) (fluorescence microscope image, nuclei were DAPI stained), alkaline phosphatase activity (F) (phase-contrast microscope image).

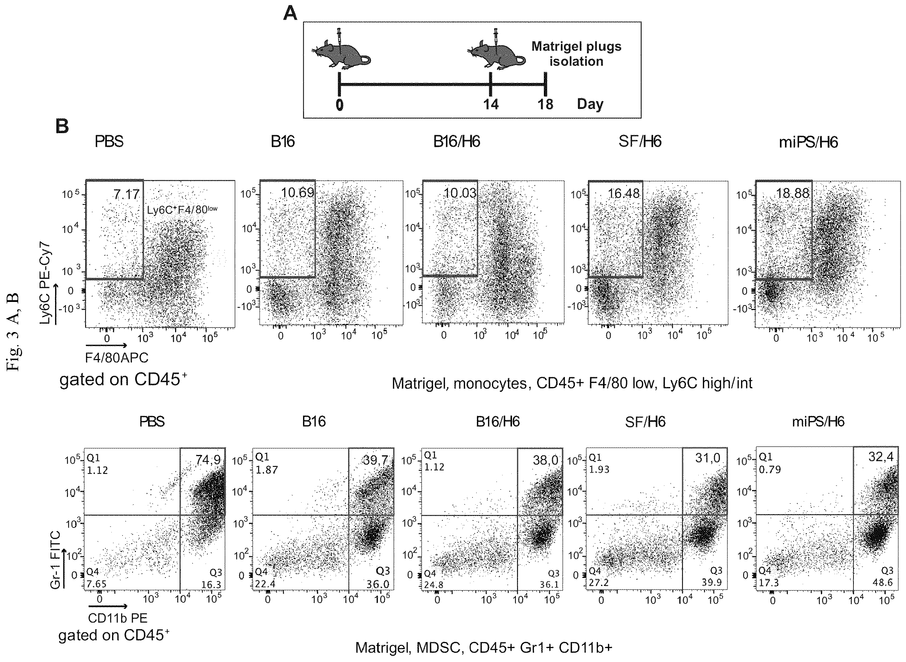

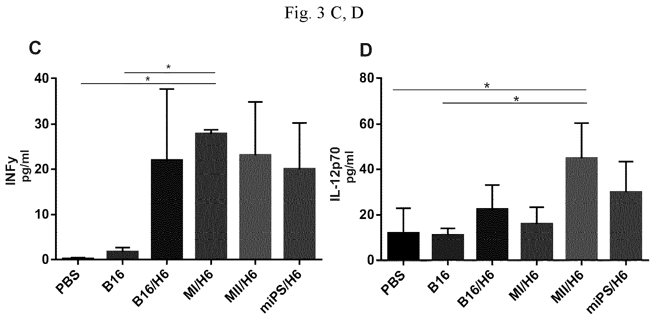

- Fig. 3: shows that cancer vaccine compositions comprising stem cells (iPSCs or sphere-derived CDSCs) are particularly effective. Mice were immunized twice as described in methods. 4 days after boosting matrigel plugs were isolated and immunological cells as well as proinflammatory cytokines were analysed (A). Immunization with SF/H6 and miPS/H6 increased the number of monocytes infiltrating the vaccine in matrigel (B) and decreased the number of MDSC (myeloid-derived suppressor cells) (B). Immunisation with SF/H6 and miPS/H6 vaccines induced local production of INFγ (C) and IL-12p70; PBS vs SF/H6 p=0.038; B16 vs SF/H6 p=0.034 (D).

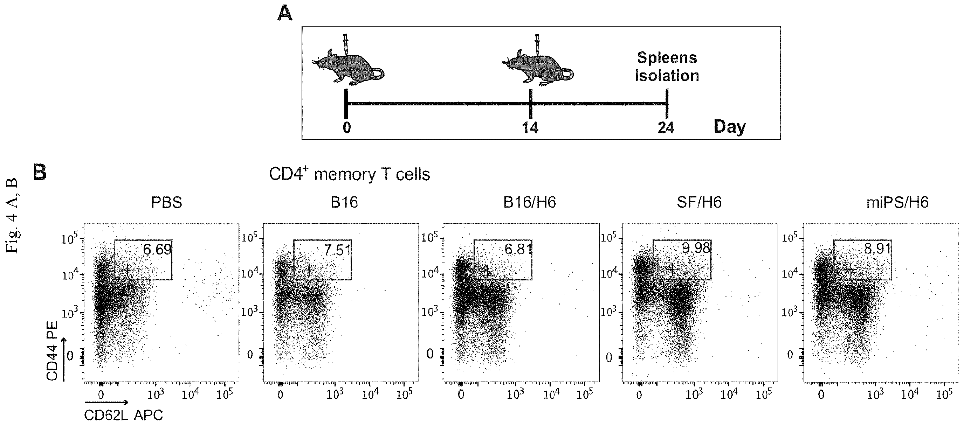

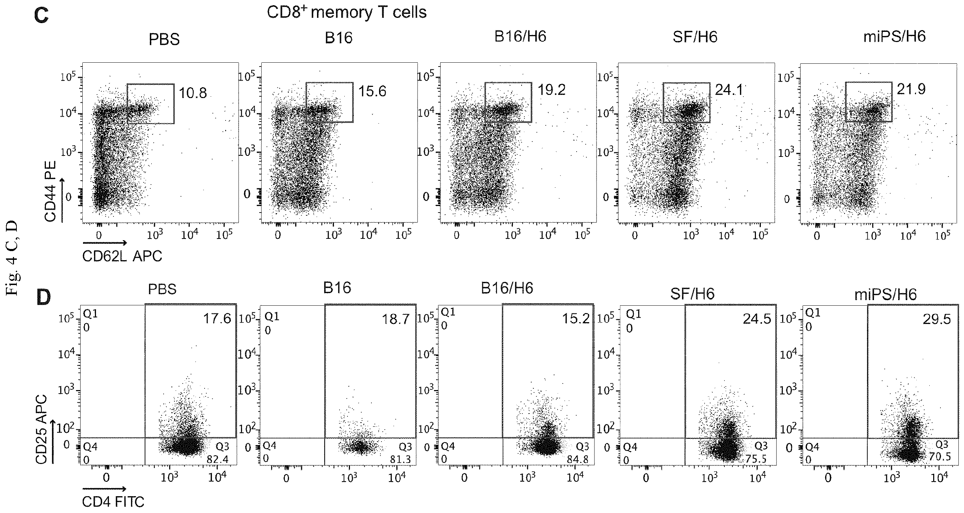

- Fig. 4: shows that cancer vaccine compositions comprising stem cells (iPSCs or sphere-derived CDSCs) are particularly effective. Mice were immunized as it was described in methods. 10 days after boosting spleens were collected. Analysis of isolated immune cells as well as cytokine release and proliferation assays have been carried out (A) Immunization with SF/H6, miPS/H6 vaccines increased CD4⁺ T cells activation (D) and the number of memory T cells in spleens (B, C).

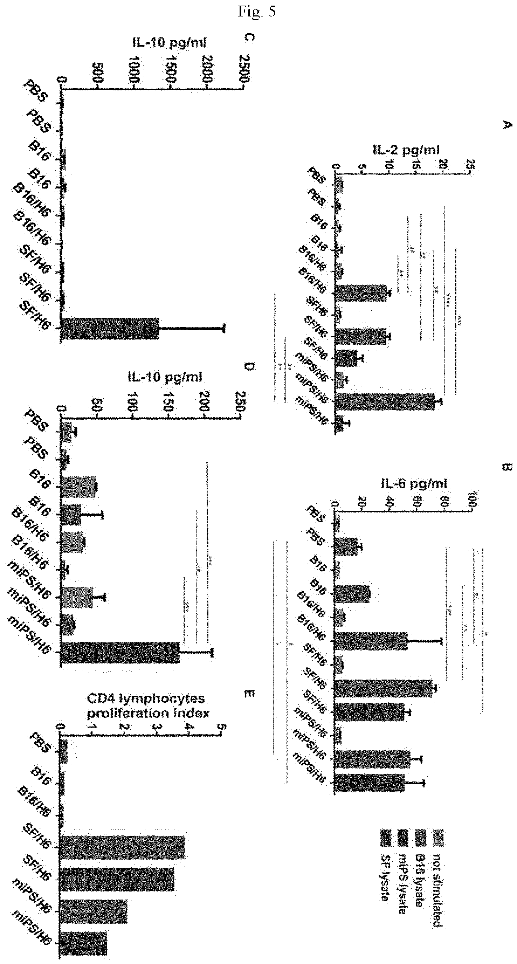

- Fig. 5: shows that immunization with SF/H6, miPS/H6 increases cytokine production by CD4+ T lymphocytes derived from spleens (A, B, C, D). Splenocytes isolated from immunized mice 10 days after priming, were in vitro re-stimulated with lysates of B16F10, SF or miPS cells. p<0.05. (E) Immunization with SF/H6, miPS/H6 increased proliferation of CD4+ lymphocytes. 3H-thymidine incorporation assay revealed increased proliferation of CD4+ lymphocytes derived from mice immunized with SF/H6, miPS/H6 vaccines compared to control vaccines.

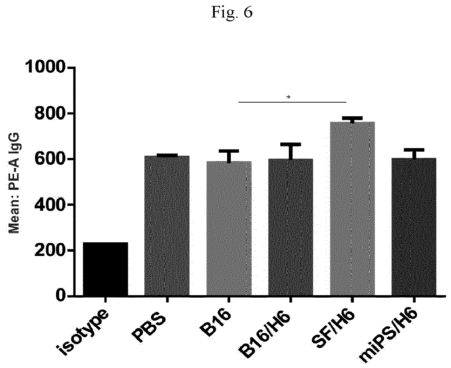

- Fig. 6: shows immunization with SF/H6 and miPS/H6 increased humoral response. B16 cells were incubated with normalized IgG level sera derived from mice 10 days after boosting, then labeled with anti-IgG PE antibody and analyzed in the cytometer. p<0.05

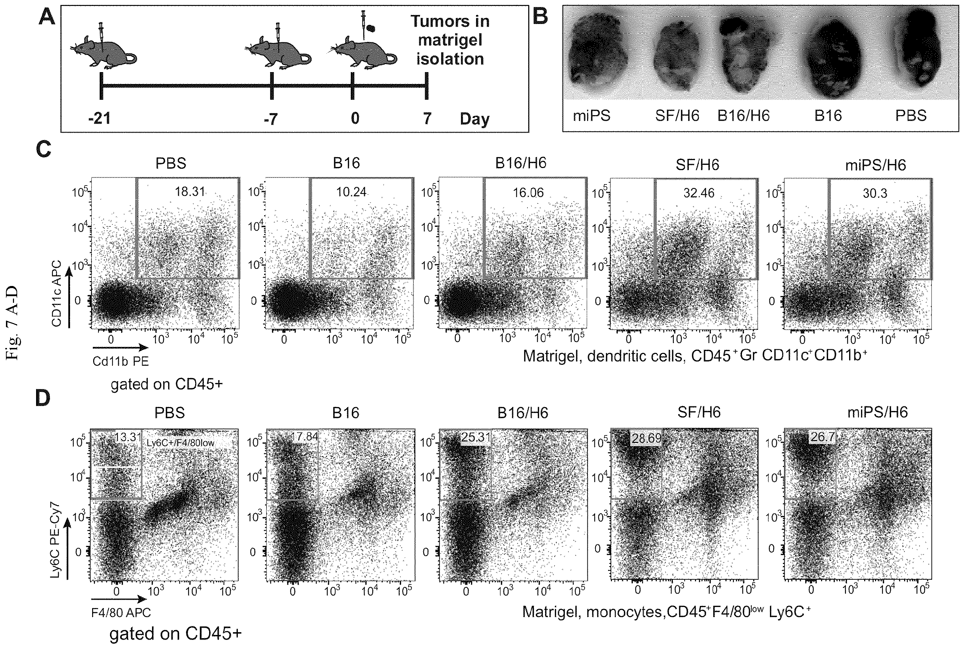

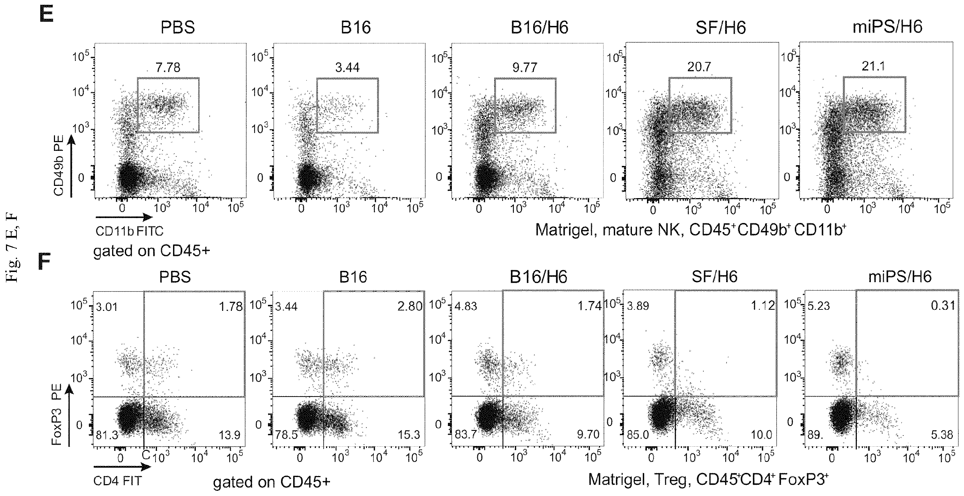

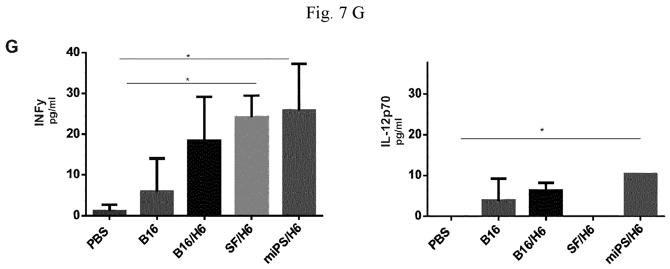

- Fig. 7: shows that cancer vaccine compositions comprising stem cells (iPSCs or sphere-derived CDSCs) are particularly effective. Mice were immunized twice as described in methods. Seven days after boosting immunisation mice were subcutaneously administered with B16F10 tumour cells in matrigel. Seven days later tumours in matrigel were excised (A) and infiltrating cells as well as proinflammatory cytokines were analyzed. Immunization with SF/H6 and miPS/H6 vaccines increased the number of tumour infiltrating dendritic cells (C) decreased the number of infiltrating monocytes (D), increased the number of infiltrating mature NK cells (E) and decreased the number of infiltrating regulatory T lymphocytes (F). Immunization with SF/H6 and miPS/H6 vaccines induced production of proinflammatory cytokines in the tumour site (G, H) p<0.5.

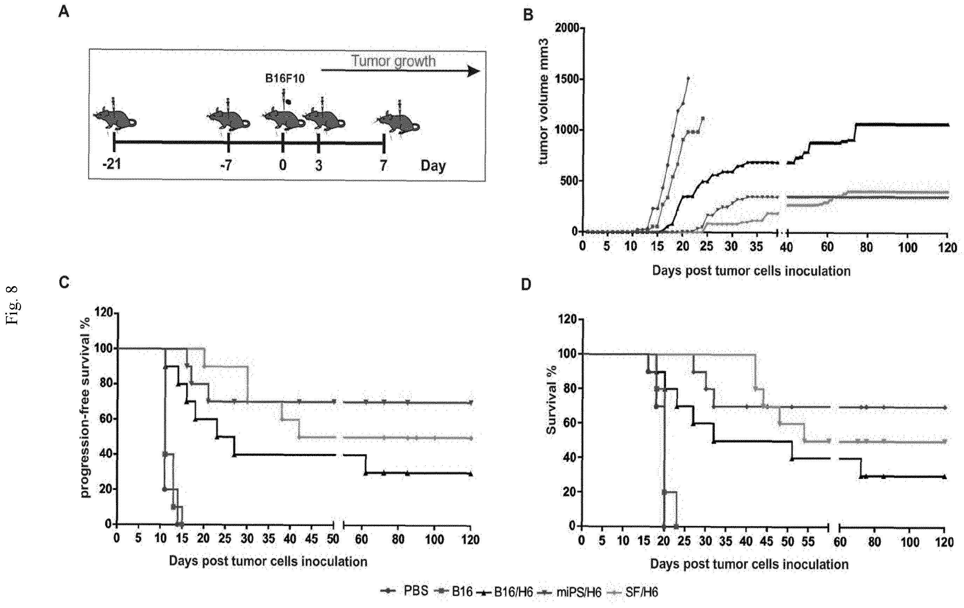

- Fig. 8: shows that cancer vaccine compositions comprising stem cells (iPSCs or sphere-derived CDSCs) are particularly effective. Mice were immunized twice. Seven days after the second immunization mice were subcutaneously administered with B16F10 tumour cells. Three and seven days later the mice were again immunized and followed up. **(A)** Immunization with SF/H6 and miPS/H6 vaccines inhibited tumour growth, extended progression-free survival and overall survival. SF/H6 and miPS/H6 vaccines inhibited tumour growth (**D21:** PBS vs B16/H6 p<0.0001; PBS vs SF/H6 p<0.0001; PBS vs miPS/H6 p<0.0001; B16 vs PBS p=0.0244; B16 vs B16/H6 p=0.004; B16 vs SF/H6 p<0.0001; B16 vs miPS/H6 p<0.0001; **D121:** B16/H6 vs SF/H6 p=0.0041; B16/H6 vs miPS/H6 p=0.0016) **(B),** extended disease free survival (PBS vs B16/H6 p=0,0001; PBS vs SF/H6 p<0.0001; PBS vs miPS/H6 p<0.0001; B16 vs B16/H6 p=0.0001, B16 vs SF/H6 p<0.0001; B16 vs miPS/H6 p<0.0001) **(C)** and overall survival (PBS vs B16/H6 p=0.001; PBS vs SF/H6 p<0.0001; PBS vs miPS/H6 p<0.0001; B16 vs B16/H6 p=0.0016; B16 vs SF/H6 p<0.0001; B16 vs miPS/H6 p<0.0001) **(D).**

### EXAMPLES

In the following, the invention is explained in more detail by the following nonlimiting examples:

### Example 1 - Construction and characterization of a murine melanoma vaccine comprising a mixture of B16F10-H6, melanospheres derived murine stem like cells (SF) or fibroblast derived inducible pluripotent stem cells (iPS).

**Cell lines and culture.** Murine B16F10 purchased from ATTC (ATCC CRL-6475™) were grown in monolayer culture in DMEM/High glucose, with stable glutamine and 10% FBS, 1 x Antibiotic/Antimycotic Solution. Mouse embryonic fibroblasts (MEF) were cultured in DMEM/High glucose, with stable glutamine, 15% FBS, 1 x Antibiotic/Antimycotic. MEF were grown, harvested, irradiated with a dose of 80 Gy (Gamma-Cell 1000, RTA), aliquoted and cryopreserved (50% FBS, 40% DMEM, 10% DMSO). miPSC were purchased from System Bioscience, sorted with anti-SSEA-1 by magnetic bead sorting and grown on inactivated MEF layer in KnockOut DMEM, 15% KnockOut Serum Replacement KSR, GIBCO), 2 mM L-glutamine, 1% Antibiotic/Antimycotic Solution, 1% Non-inactivated Essential Amino-acids and 1000U/ml LIF (leukemia inhibitory factor). MiPSC were passaged every 2-3 days using 1 x TrypLE Select Enzyme and elimination of MEF by selective incubation on 0,1% gelatin coated plate. MiPSC were cultured under conditions maintaining their pluripotency, irradiated with 80 Gy, ampuled and cryopreserved (50%).

**Transduction of B16F10 cells with H6 cDNA.** E1-depleted adenoviral recombinant of human strain 5 encoding H6 (AdH6) was constructed, propagated and titrated on E1-transfected 293 cells (64). B16F10 cells at 90% confluence were transduced using AdH6 by 24h incubation. Then cells (B16H6) were harvested, irradiated (80 Gy), aliquoted and stored in liquid nitrogen.

**Melanospheres.** B16F10 cells were cultured under conditions inducing melanospheres formation in DMEM/F12 medium with GlutaMAX, 2% B27 supplement, 1% antibiotic/antimycotics. Immediately before use recombinant basic Fibroblast Growth Factor (bFGF) and recombinant Epidermal Growth Factor (EGF) were added to the final concentration of 20ng/ml and 10ng/ml, respectively (65, 66). Melanospheres were cultured for 10 weeks and passaged weekly. At each passage expression of Mitf, tyrosinase, Nanog, VEGF, STAT3 phosphorylation and the ALDH1 activity were analyzed.

**Melanosphere cells demonstrated melanoma stem cells-like phenotype.** Cells obtained from spheres demonstrated significantly increased Stat3 phosphorylation, increased activity of ALDH1 (10-fold), increased expression of VEGFa, CD44 and PD-L1, while decreased tyrosinase, MITF and MHC compared to B16F10 cells cultured as monolayers (Fig. 1).

**Phenotypic analysis of miPS.** The Mouse Pluripotent Stem Cell Analysis Kit was used for cytometric analyzes. Immunocytochemistry staining was performed to verify the parent cell phenotype using anti-SSEA-1, anti-Epcam, anti-E-cadherin, anti-Nanog antibodies. MiPS cultured in conditions supporting pluripotency expressed SSEA-1, Epcam, E-cadherin, NANOG and alkaline phosphatase - early marker of pluripotency (Fig. 2a-e). Real-time PCR demonstrated higher expression of NANOG, Oct 3/4 and SSEA-1 in miPS than embryonic stem cells (Fig. 2f)

**Determination of aldehyde dehydrogenase activity - ALDH.** ALDH activity was investigated using the ALDEFLUOR™ Kit.

**Vaccine composition.** Three therapeutic vaccines and 2 controls were constructed. SF/H6 vaccine consisted of 1x10⁶ cells obtained from melanospheres admixed with 5x10⁵ B16H6 cells. miPS/H6 vaccine - consisted of 1x10⁶ miPS admixed with 5x10⁵ B16H6 cells. B16/H6 - consisted of 1x10⁶ wild type B16F10 cells, admixed with 5x10⁵ B16H6 cells; B16 - control consisted of 1,5x10⁶ wild B16F10 cells, PBS - control consisted of 200 µl of PBS buffer. All cells were irradiated with a dose of 80 Gy.

### Example 2 - Immunotherapy of murine melanoma with irradiated B 16H6/SF and B 16H6/iPS vaccines.

**Mice.** 5-6 week old C57BL/6 male mice were purchased from Charles River Laboratories (Sulzfeld, Germany). Mice were maintained under constant pathogen-free conditions and maintained a 12-hour day/night cycle with unrestricted access to food and water. The experiments were initiated after the two-week quarantine. All experiments were carried out at least twice in accordance with the national and institutional guidelines for human treatment of laboratory animals, after obtaining the consent of the Local Ethics Committee for Experiments on Animals in Poznań (No. 67/2012).

**Clinical effect of immunization with the vaccines tested.** In the prophylactic model, a significant delay in tumour take was observed in mice immunized with SF/H6 and miPS/H6 vaccines. Moreover, significant extension of DFS and OS were seen. In the control groups (PBS, B16), tumours appeared 11 days after sc. administration of non-irradiated, wild type B16F10 cells. (Fig. 8b, c), reached a maximum size after 20 days (Fig. 8a), and were euthanized due to the volume. In mice immunized with B16/H6, first tumours appeared 11 days after administration of B16F10 cells (Fig. 8b, c), while tumour formation of was delayed as compared to B16 and PBS immunized mice (Fig. 8b). Median survival of the above group of mice was 41.5 days (Fig. 8d). 30% of animals survived until the end of experiment. In mice immunized with **MI/H6** tumours started to appear 16 days after administration of B16F10 cells (Fig. 8b, c). Their growth kinetics ware slower compared to the control groups and B16/H6. (Fig. 8b) The median survival was 85.5 days and 50% of mice survived to the end of the experiment (Fig 8d). In animals immunized with SF/H6, the first tumours appeared 20 days after B16F10 administration (Fig. 8b, c). Their growth kinetics were much slower than in the control groups (B16, PBS) and B16/H6 (Fig. 8b). Median survival time was 87 days and 50% of mice survived to the end of experiment. In mice immunized with the miPS/H6 vaccine, the first tumours appeared 16 days after B16F10 administration (Fig. 8b, c). A significant delay in the tumour take was observed compared to the control groups and B16/H6 (Fig. 8b). Animals immunized with miPS/H6 lived significantly longer than control mice immunized with B16/H6 or and SF/H6. 70% of mice survived until the end of the experiment (Fig. 8d).

### Example 3 - Analysis of the immune response induced by the vaccine.

### A. Immune response at the site of vaccine administration and spleens

Mice (5-7 animals per group) were immunized sc. twice in two weeks intervals. Cells of the first dose were suspended in PBS (250 µ1), while of the second dose in matrigel (250 µ1) (BD-Matrigel®Matrix BasementMembrane High Concentration). Matrigel "tumours" were resected 4 days later (Fig. 3a), cut into pieces and incubated at 37°C for 30 min with a mix of collagenase (1mg/ml, Roche), DNase (0.3 mg/ml, Roche) and dispase (50U/ml, Corning). Digested material was cooled on ice, passed through Falcon® 70µm Cell Strainer and centrifuged. Supernatants were collected and stored at -80°C for further cytokine analyses. Cells were then washed in PBS, counted, stained with monoclonal antibodies and analyzed with flow cytometry. Proinflammatory cytokines (IL-12p70, INFy) concentrations were analyzed using Cytometric Bead Array (CBA) mouse inflammation kit (BD Biosciences) according to a manufacturer protocol.
Ten days after the second dose administration, spleens from each study subgroup were removed, pooled (Fig. 4a), minced and drained through a Cell Strainer 70µM (BD). The relative amounts (%) of T CD4 +, T CD8 +, memory cells, myeloid derived suppressor cells (MDSC) and CD4 + Foxp3 + regulatory T cells (Treg) in spleens were assessed and compared.

**Cellular infiltrates and cytokines in the vaccination site and spleens.** In the site of vaccine administration there was increased number of inflammatory monocytes infiltrating vaccine cells of SF/H6 and miPS/H6 immunized mice as compared to B16/H6 and control mice (Fig. 3b). In contrary, there was decreased number of MDSC in corresponding experimental groups (Fig. 3b). There was dramatic increase of INFγ in immunized mice compared to controls. There were no statistically significant differences between vaccine variants. There was also significant increase of IL-12p70 in mice immunized with SF/H6 (3-4 fold) and miPS/H6 (2.5 fold) as compared with controls (Fig. 3c, d).

In spleens collected 10 days following immunization significant increase of activated T CD4⁺ cells was observed in SF/H6 and miPS/H6 immunized mice compred to B16/H6 and control mice (Fig. 4d). The highest T CD4⁺ activation was seen for miPS/H6. In the same groups of mice increased memory T CD4⁺ cells number was seen (Fig. 4b). In SF/H6 and miPS/H6 immunized mice increased number of memory T CD8⁺ cells was observed as compared to control mice. In mice immunized with B16/H6, memory T CD8⁺ cells number was between control and stem cells vaccine groups (Fig. 4c).

**T helper cytokine release and proliferation assays.** Cells isolated from spleens were seeded into 96-well plates in concentration of 1×10⁶ per well and were re-stimulated with lysates of B16 or SF cell (SF/H6 study group) and miPS cells (miPS/H6 study group) at ratio 2:1. Positive controls were polyclonally stimulated with anti-CD3/CD28 antibodies, while negative controls were non-stimulated. After 72 hours 50 µl medium was harvested for cytokine analysis and replaced with 50 µl of x-vivo medium with 3H-thymidine at 1 µC per well for culture continuation. After 18 hours cells were harvested and the lymphocytes proliferation was measured using a β scintillation counter (1450 Luminescence Counter Micro Beta TriLux). The concentration of cytokines was analyzed using the Mouse Th1/Th2/Th17 Cytokine Cytometric Bead Array kit (BD).

**Antigen specific T CD4⁺ responses.** Antigen specific T CD4⁺ lymphocytes upon restimulation with B16, melanosphers (SF) and miPS lysates secreted cytokines. On restimulation with B16 cells lysate, approximately 20-fold increase of IL-2 production was recorded in mice immunized with miPS/H6, 10-fold with SF/H6 and B16/H6 relative to PBS control. However, in this group of mice no IL-2 secretion was seen upon restimulation with miPS cells lysate (Fig. 5a). In the miPS/H6 immunized group, miPSC lysate re-stimulation caused 20-fold increase in IL-10 production as compared to PBS and B16/H6 (Fig. 5d) Higher production of IL-10 was also observed in mice immunized with SF/H6, when splenocytes were re-stimulated with lysates from melanospheres (Fig. 5c). No IL-10 was secreted upon restimulation in B 16/H6 group. Increased IL-6 production was also observed in animals immunized with B16/H6 (2-3 fold), SF/H6 (3-4 fold) and miPS/H6 (2-3 fold) after re-stimulation with vaccine lysates and B16 (Fig. 5b). Increased proliferation of CD4 + T helper lymphocytes from spleens of immunized mice in response to re-stimulation with B16, SF or miPS cell lysate were found. In the culture of splenocytes from mice immunized with SF/H6 both at re-stimulation with B16 and SF, 20-30 times higher proliferation coefficient was observed compared to control and immunized with B16/H6 mice. In mice immunized with the miPS/H6 vaccine after re-stimulation with B16 or miPS lysates, the proliferation index was approximately 10-20 times higher than in the control groups. In the SF/H6 immunized group, re-stimulation resulted in 8-15 fold higher proliferation index as compared to the control groups and B16H6 (Fig. 5e)

### B. Immune response in the tumour microenvironment.

**Analysis of tumour-infiltrating immune cells.** In order to analyze cells infiltrating tumours, mice were immunized twice with an interval of 14 days. Seven days after second immunization mice were re-challenged s.c. with 1x10⁵ B16F10 cells suspended in 250 µl of matrigel. Three days after, mice were again immunized and seven days after B16F10 injection tumours in matrigel were excised. Infiltrating cells were recovered by enzymatic digestion with collagenase (1mg/ml, Roche), DNaze (0.3 mg/ml, Roche) and dispaze (50 U/ml, Corning) as described previously. Cells were then washed in PBS, counted and stained with monoclonal antibodies.

**Effector immune response.** In Fig. 7b the matrigel tumours immediately after excision before isolation of infiltrating cells are shown. In the matrigels removed from the control (PBS, B16), unlike the immunized groups B16/H6, SF/H6 and miPS/H6, clearly formed tumours are visible. Analysis of infiltrating cells showed about 1.5-2 times lower percentage of CD Foxp3 Tregs cells in tumours isolated from mice immunized with SF/H6 compared to immunized with B16/H6 and about 2-3-fold lower compared to the immunized with irradiated B16F10 cells or PBS. Especially reduced percentage of infiltrating Tregs was observed in mice immunized with miPS/H6, which was about 5.5 fold lower than in the B16/H6 group and 9 times lower compared to the groups immunized with B16F10 cells and PBS (Fig. 7f).

A significantly higher number of NK cells in the tumour infiltrates of immunized groups SF/H6, and miPS/H6 were observed. It was about 6 times higher than in the group of B16F10 vaccinated mice and about 2-3 times higher than in groups receiving B16/H6 (Fig. 7e).

In addition, in tumours collected from mice immunized with SF/H6 and miPS/H6, 2-3 fold higher percentage of monocytes as compared to B16F10 and PBS groups and about 1.5 times higher than B16/H6 group was found (Fig. 7d). In tumour infiltrates of MI/H6-vaccinated mice the percentage of monocytes was also higher than in the PBS and B16F10 groups, however, lower than in B16/H6 group (Fig. 7d). Relative amount of dendritic cells (DC) infiltrating tumours was 2-fold higher in SF/H6 and miPS/H6 than in B16/H6 (Fig. 7c).

Highest and significant production of INFγ in the tumour microenvironment of mice immunized with SF/H6 and miPS/H6 was found, while highest and significant IL-12p70 in miPS/H6 vaccinated (Fig. 7g, h).

### C. Humoral response in immunized mice.

There was an increased (statistically significant) amount of antibodies directed to B16F10 melanoma cell antigens in serum from mice immunized with SF/H6 10 days after the two weeks priming with vaccines (Fig. 6).

**Table 1. Deposit numbers of cell lines used**

| Name of cell line | International Depository Authority | Depositor | Date of Deposit | Accession number given by the International Depository Authority |
|---|---|---|---|---|
| Mich1 | DSMZ¹ | AGIRx ² | 24 April 2007 | DSM ACC2837 |
| Mich2 | DSMZ | AGIRx | 24 April 2007 | DSM ACC2838 |
| Mich1-H6 | DSMZ | AGIRx | 24 April 2007 | DSM ACC2839 |
| Mich2-H6 | DSMZ | AGIRx | 24 April 2007 | DSM ACC2840 |

| | | | | |
|---|---|---|---|---|
| ¹ DSMZ - Deutsche Sammlung von Mikroorganismen und Zellkulturen GmbH, Inhoffenstr. 7 B, D-38124 Braunschweig, Germany ² AGIRx (Active Gene Interventions) Ltd., 1 The Courtyard, Chalvington, East Sussex BN273TD, UK | | | | |

### REFERENCES

1. Schreiber RD, Old LJ, Smyth MJ. Cancer immunoediting: integrating immunity's roles in cancer suppression and promotion. Science (New York, NY). 2011;331(6024):1565-70.
2. Kortylewski M, Yu H. Role of Stat3 in suppressing anti-tumor immunity. Current opinion in immunology. 2008;20(2):228-33.
3. Vesely MD, Kershaw MH, Schreiber RD, Smyth MJ. Natural innate and adaptive immunity to cancer. Annual review of immunology. 2011;29:235-71.
4. Visvader JE, Lindeman GJ. Cancer stem cells: current status and evolving complexities. Cell stem cell. 2012;10(6):717-28.
5. Kubler H, Vieweg J. Vaccines in renal cell carcinoma. Seminars in oncology. 2006;33(5): 614-24.
6. Mach N, Dranoff G. Cytokine-secreting tumor cell vaccines. Current opinion in immunology. 2000;12(5):571-5.
7. Ward S, Casey D, Labarthe MC, Whelan M, Dalgleish A, Pandha H, et al. Immunotherapeutic potential of whole tumour cells. Cancer immunology, immunotherapy : CII. 2002;51(7):351-7.
8. Mackiewicz A, Mackiewicz J, Wysocki PJ, Wiznerowicz M, Kapcinska M, Laciak M, et al. Long-term survival of high-risk melanoma patients immunized with a Hyper-IL-6-modified allogeneic whole-cell vaccine after complete resection. Expert opinion on investigational drugs. 2012;21(6):773-83.
9. Kozlowska A, Mackiewicz J, Mackiewicz A. Therapeutic gene modified cell based cancer vaccines. Gene. 2013;525(2):200-7.
10. Scheffer SR, Nave H, Korangy F, Schlote K, Pabst R, Jaffee EM, et al. Apoptotic, but not necrotic, tumor cell vaccines induce a potent immune response in vivo. International journal of cancer. 2003;103(2):205-11.
11. Porgador A, Tzehoval E, Katz A, Vadai E, Revel M, Feldman M, et al. Interleukin 6 gene transfection into Lewis lung carcinoma tumor cells suppresses the malignant phenotype and confers immunotherapeutic competence against parental metastatic cells. Cancer research. 1992;52(13):3679-86.
12. Ledda MF, Adris S, Bravo AI, Bover L, Carbone C, Paleolog E, et al. Tumor cells engineered to express interleukin-6 exhibit a reduced tumorigenicity depending on the tumor cell model. Cellular and molecular biology (Noisy-le-Grand, France). 1996;42(5):769-78.
13. Wysocki PJ, Kowalczyk DW, Izycki D, Grabarczyk P, Kwias Z, Mackiewicz A. IL-6 and GM-CSF in tumor rejection model of renal cell cancer. Advances in experimental medicine and biology. 2001;495:379-83.
14. Taga T, Kishimoto T. Gp130 and the interleukin-6 family of cytokines. Annual review of immunology. 1997;15:797-819.
15. Mackiewicz A, Schooltink H, Heinrich PC, Rose-John S. Complex of soluble human IL-6-receptor/IL-6 up-regulates expression of acute-phase proteins. Journal of immunology (Baltimore, Md : 1950). 1992;149(6):2021-7.
16. Weissman IL. Stem cells: units of development, units of regeneration, and units in evolution. Cell. 2000;100(1):157-68.
17. Denker HW. Stem cell terminology and 'synthetic' embryos: a new debate on totipotency, omnipotency, and pluripotency and how it relates to recent experimental data. Cells, tissues, organs. 2014;199(4):221-7.
18. Condic ML. Totipotency: what it is and what it is not. Stem cells and development. 2014;23(8):796-812.
19. De Paepe C, Krivega M, Cauffman G, Geens M, Van de Velde H. Totipotency and lineage segregation in the human embryo. Molecular human reproduction. 2014;20(7):599-618.
20. Dulak J, Szade K, Szade A, Nowak W, Jozkowicz A. Adult stem cells: hopes and hypes of regenerative medicine. Acta biochimica Polonica. 2015;62(3):329-37.
21. Thomson JA, Itskovitz-Eldor J, Shapiro SS, Waknitz MA, Swiergiel JJ, Marshall VS, et al. Embryonic stem cell lines derived from human blastocysts. Science (New York, NY). 1998;282(5391): 1145-7.
22. Stenudd M, Sabelstrom H, Frisen J. Role of endogenous neural stem cells in spinal cord injury and repair. JAMA neurology. 2015;72(2):235-7.
23. Dzierzak E, Speck NA. Of lineage and legacy: the development of mammalian hematopoietic stem cells. Nature immunology. 2008;9(2): 129-36.
24. Kfoury Y, Scadden DT. Mesenchymal cell contributions to the stem cell niche. Cell stem cell. 2015;16(3):239-53.
25. Dumont NA, Bentzinger CF, Sincennes MC, Rudnicki MA. Satellite Cells and Skeletal Muscle Regeneration. Comprehensive Physiology. 2015;5(3):1027-59.
26. Takahashi K, Tanabe K, Ohnuki M, Narita M, Ichisaka T, Tomoda K, et al. Induction of pluripotent stem cells from adult human fibroblasts by defined factors. Cell. 2007;131(5):861-72.
27. Puri MC, Nagy A. Concise review: Embryonic stem cells versus induced pluripotent stem cells: the game is on. Stem cells (Dayton, Ohio). 2012;30(1):10-4.
28. Guenther MG, Frampton GM, Soldner F, Hockemeyer D, Mitalipova M, Jaenisch R, et al. Chromatin structure and gene expression programs of human embryonic and induced pluripotent stem cells. Cell stem cell. 2010;7(2):249-57.
29. Takahashi K, Yamanaka S. Induction of pluripotent stem cells from mouse embryonic and adult fibroblast cultures by defined factors. Cell. 2006;126(4):663-76.
30. Kaji K, Norrby K, Paca A, Mileikovsky M, Mohseni P, Woltjen K. Virus-free induction of pluripotency and subsequent excision of reprogramming factors. Nature. 2009;458(7239):771-5.
31. Sommer CA, Stadtfeld M, Murphy GJ, Hochedlinger K, Kotton DN, Mostoslavsky G. Induced pluripotent stem cell generation using a single lentiviral stem cell cassette. Stem cells (Dayton, Ohio). 2009;27(3):543-9.
32. Zhou W, Freed CR. Adenoviral gene delivery can reprogram human fibroblasts to induced pluripotent stem cells. Stem cells (Dayton, Ohio). 2009;27(11):2667-74.
33. Baum CM, Weissman IL, Tsukamoto AS, Buckle AM, Peault B. Isolation of a candidate human hematopoietic stem-cell population. Proceedings of the National Academy of Sciences of the United States of America. 1992;89(7):2804-8.
34. Bonnet D, Dick JE. Human acute myeloid leukemia is organized as a hierarchy that originates from a primitive hematopoietic cell. Nature medicine. 1997;3(7):730-7.
35. Singh SK, Hawkins C, Clarke ID, Squire JA, Bayani J, Hide T, et al. Identification of human brain tumour initiating cells. Nature. 2004;432(7015):396-401.
36. Al-Hajj M, Wicha MS, Benito-Hernandez A, Morrison SJ, Clarke MF. Prospective identification of tumorigenic breast cancer cells. Proceedings of the National Academy of Sciences of the United States of America. 2003;100(7):3983-8.
37. Prince ME, Sivanandan R, Kaczorowski A, Wolf GT, Kaplan MJ, Dalerba P, et al. Identification of a subpopulation of cells with cancer stem cell properties in head and neck squamous cell carcinoma. Proceedings of the National Academy of Sciences of the United States of America. 2007;104(3):973-8.
38. Yang ZF, Ho DW, Ng MN, Lau CK, Yu WC, Ngai P, et al. Significance of CD90+ cancer stem cells in human liver cancer. Cancer cell. 2008;13(2):153-66.
39. Hermann PC, Huber SL, Herrler T, Aicher A, Ellwart JW, Guba M, et al. Distinct populations of cancer stem cells determine tumor growth and metastatic activity in human pancreatic cancer. Cell stem cell. 2007;1(3):313-23.
40. Dalerba P, Dylla SJ, Park IK, Liu R, Wang X, Cho RW, et al. Phenotypic characterization of human colorectal cancer stem cells. Proceedings of the National Academy of Sciences of the United States of America. 2007;104(24):10158-63.
41. Chan KS, Espinosa I, Chao M, Wong D, Ailles L, Diehn M, et al. Identification, molecular characterization, clinical prognosis, and therapeutic targeting of human bladder tumor-initiating cells. Proceedings of the National Academy of Sciences of the United States of America. 2009;106(33): 14016-21.
42. Collins AT, Berry PA, Hyde C, Stower MJ, Maitland NJ. Prospective identification of tumorigenic prostate cancer stem cells. Cancer research. 2005;65(23): 10946-51.
43. Fang D, Nguyen TK, Leishear K, Finko R, Kulp AN, Hotz S, et al. A tumorigenic subpopulation with stem cell properties in melanomas. Cancer research. 2005;65(20):9328-37.
44. Steg AD, Bevis KS, Katre AA, Ziebarth A, Dobbin ZC, Alvarez RD, et al. Stem cell pathways contribute to clinical chemoresistance in ovarian cancer. Clinical cancer research : an official journal of the American Association for Cancer Research. 2012;18(3):869-81.
45. Bose D, Zimmerman LJ, Pierobon M, Petricoin E, Tozzi F, Parikh A, et al. Chemoresistant colorectal cancer cells and cancer stem cells mediate growth and survival of bystander cells. British journal of cancer. 2011;105(11):1759-67.
46. Beier D, Schulz JB, Beier CP. Chemoresistance of glioblastoma cancer stem cells-much more complex than expected. Molecular cancer. 2011;10:128.
47. Clevers H. The cancer stem cell: premises, promises and challenges. Nature medicine. 2011;17(3):313-9.
48. Waldron NN, Kaufman DS, Oh S, Inde Z, Hexum MK, Ohlfest JR, et al. Targeting tumor-initiating cancer cells with dCD133KDEL shows impressive tumor reductions in a xenotransplant model of human head and neck cancer. Molecular cancer therapeutics. 2011; 10(10): 1829-3 8.
49. Hilton J. Role of aldehyde dehydrogenase in cyclophosphamide-resistant L1210 leukemia. Cancer research. 1984;44(11):5156-60.
50. Abdullah LN, Chow EK. Mechanisms of chemoresistance in cancer stem cells. Clinical and translational medicine. 2013;2(1):3.
51. Fischer M, Goldschmitt J, Peschel C, Brakenhoff JP, Kallen KJ, Wollmer A, et al. I. A bioactive designer cytokine for human hematopoietic progenitor cell expansion. Nature biotechnology. 1997;15(2):142-5.
52. Rose-John S, Schooltink H, Lenz D, Hipp E, Dufhues G, Schmitz H, et al. Studies on the structure and regulation of the human hepatic interleukin-6 receptor. European journal of biochemistry. 1990;190(1):79-83.
53. Kim SY, Kang JW, Song X, Kim BK, Yoo YD, Kwon YT, et al. Role of the IL-6-JAK1-STAT3-Oct-4 pathway in the conversion of non-stem cancer cells into cancer stem-like cells. Cellular signalling. 2013;25(4):961-9.
54. Coulie PG. Antigens recognized on human tumors by cytolytic T lymphocytes: towards vaccination? Stem cells (Dayton, Ohio). 1995;13(4):393-403.
55. Malicki J, Laciak M, Kosicka G, Kierzkowski J, Wiznerowicz M, Mackiewicz A. Effect of irradiation on interleukin 6 and soluble interleukin 6 receptor modified melanoma genetic vaccine. Reports of Practical Oncology. 1996; 1(2): 104-9.
56. Nawrocki S, Murawa P, Malicki J, Kapcinska M, Gryska K, Izycki D, et al. Genetically modified tumour vaccines (GMTV) in melanoma clinical trials. Immunology letters. 2000;74(1):81-6.
57. Drakesmith H, O'Neil D, Schneider SC, Binks M, Medd P, Sercarz E, et al. In vivo priming of T cells against cryptic determinants by dendritic cells exposed to interleukin 6 and native antigen. Proceedings of the National Academy of Sciences of the United States of America. 1998;95(25): 14903-8.
58. Dominitzki S, Fantini MC, Neufert C, Nikolaev A, Galle PR, Scheller J, et al. Cutting edge: trans-signaling via the soluble IL-6R abrogates the induction of FoxP3 in naive CD4+CD25 T cells. Journal of immunology (Baltimore, Md : 1950). 2007;179(4):2041-5.
59. Ozbek S, Peters M, Breuhahn K, Mann A, Blessing M, Fischer M, et al. The designer cytokine hyper-IL-6 mediates growth inhibition and GM-CSF-dependent rejection of B16 melanoma cells. Oncogene. 2001;20(8):972-9.
60. Kwiatkowska-Borowczyk E, Czerwinska P, Mackiewicz J, Gryska K, Kazimierczak U, Tomela K, et al. Whole cell melanoma vaccine genetically modified to stem cells like phenotype generates specific immune responses to ALDH1A1 and long-term survival in advanced melanoma patients. Oncoimmunology. 2018;7(11):e1509821.
61. Czerwinska P, Rucinski M, Wlodarczyk N, Jaworska A, Grzadzielewska I, Gryska K, et al. Therapeutic melanoma vaccine with cancer stem cell phenotype represses exhaustion and maintains antigen-specific T cell stemness by up-regulating BCL6. Oncoimmunology. 2020;9(1): 1710063.
62. Joseph Sambrook F, E. F., Maniatis T. Molecular cloning a laboratory manual 2nd ed. 2nd ed: Cold Spring Harbor Laboratory in Cold Spring Harbor, N.Y 1989.
63. Leuenberger HGW, Nagel B, Kölbl H. A multilingual glossary of biotechnological terms (IUPAC recommendations): in English, French, German, Japanese, Portuguese, Russian, and Spanish: Verlag Helvetica Chimica Acta; 1995.
64. Kowalczyk DW, Wlazlo AP, Shane S, Ertl HC. Vaccine regimen for prevention of sexually transmitted infections with human papillomavirus type 16. Vaccine. 2001;19(25-26):3583-90.
65. Mo J, Sun B, Zhao X, Gu Q, Dong X, Liu Z, et al. The in-vitro spheroid culture induces a more highly differentiated but tumorigenic population from melanoma cell lines. Melanoma research. 2013;23(4):254-63.
66. Galli R, Binda E, Orfanelli U, Cipelletti B, Gritti A, De Vitis S, et al. Isolation and characterization of tumorigenic, stem-like neural precursors from human glioblastoma. Cancer research. 2004;64(19):7011-21.

## Claims

1. A cancer vaccine composition comprising
a. one or more first cancer cells modified to express a first hyper-cytokine,
b. optionally one or more second cancer cells modified to express a second hyper-cytokine, and
c. one or more stem cells,
wherein the first and/or second hyper-cytokine is a fusion protein comprising a cytokine receptor and a cytokine.

2. The composition according to claim 1, wherein the stem cells are induced pluripotent stem cells (iPSCs) or cancer cell derived stem cells (CDSCs), wherein optionally the stem cells are modified to express a third hyper-cytokine.

3. The composition according to claim 2, wherein the CDSCs are obtained by inducing stem cell like properties in one or more third cancer cells, in particular by cultivating the third cancer cells in a sphere culture.

4. The composition according to any of claims 1 to 3, wherein the first, second and/or third cancer cells are independently selected from a melanoma cell, a renal carcinoma cell, a prostate cancer cell, a colon cancer cell, a lung cancer cell, a pancreas cancer cell, a liver cancer cell, a brain cancer cell, a head and neck cancer cell, or a sarcoma cell.

5. The composition according to any of claims 3 to 4, wherein the CDSCs are **characterized by** at least one of: increased Stat3 phosphorylation, increased ALDH1 activity, increased expression of VEGF, CD44 and/or PD-L1 and decreased expression of tyrosinase, MITF and/or MHC in comparison to said third cancer cells, particularly the CDSCs are **characterized by** increased Stat3 phosphorylation, increased ALDH1 activity, increased expression of VEGF, CD44 and PD-L1 and decreased expression of tyrosinase, MITF and/or MHC in comparison to said third cancer cells, more particularly the CDSCs are obtained by inducing stem cell like properties in melanoma cells and are **characterized by** increased Stat3 phosphorylation, increased ALDH1 activity, increased expression of VEGF, CD44 and PD-L1 and decreased expression of tyrosinase, MITF and MHC in comparison to said melanoma cells.

6. The composition according to claim 2, wherein the iPSCs are **characterized by** expression of at least one of the following markers: SSEA-1, Epcam, E-cadherin, NANOG, alkaline phosphatase and/or Oct3/4, particularly by expression of Epcam, E-cadherin, NANOG, alkaline phosphatase and Oct3/4, more particularly by expression of SSEA-1, Epcam, E-cadherin, NANOG, alkaline phosphatase and Oct3/4.

7. The composition according to any of claims 1 to 6, wherein the first, second and/or third hyper-cytokine is a fusion protein comprising
a. an IL-6 receptor (IL-6R) part exhibiting at least 90% sequence identity to human soluble IL-6R (sIL-6R), wherein said sequence identity is calculated over the entire length of the polypeptide sequence from P113 to A323 of SEQ ID NO: 1,
b. an IL-6 part exhibiting at least 90% sequence identity to human interleukin-6 (IL-6), wherein said sequence identity is calculated over the entire length of the polypeptide sequence from P29 to M212 of SEQ ID NO: 2, and
c. optionally a peptide linker;
having hyper-cytokine activity or
a. an IL-11 receptor (IL-11R) part exhibiting at least 90% sequence identity to human soluble IL-11 receptor (sIL-11R), wherein said sequence identity is calculated over the entire length of the polypeptide sequence from M1 to Q365 of SEQ ID NO: 3,
b. an IL-11 part exhibiting at least 90% sequence identity to human interleukin-11 (IL-11), wherein said sequence identity is calculated over the entire length of the polypeptide sequence from A19 to L199 of SEQ ID NO: 4, and
c. optionally a peptide linker
having hyper-cytokine activity.

8. The composition according to any of claims 1 to 7, wherein the first, second and/or third hyper-cytokine comprises or consists of
a. a polypeptide having the amino acid sequence according to SEQ ID NO: 5, SEQ ID NO: 6, SEQ ID NO: 7, SEQ ID NO: 8 or SEQ ID NO: 9; or
b. a polypeptide exhibiting at least 90% sequence identity to a polypeptide according to (a) and having hyper-cytokine activity.

9. The composition according to any of claims 1 to 8, wherein the first and/or second cancer cells modified to express a first or second hyper-cytokine are melanoma cells, in particular Michl-H6, deposited under accession number DSM ACC2839 with DSMZ, or Mich2-H6, deposited under accession number DSM ACC2840 with DSMZ

10. The composition according to any of claims 1 to 9, wherein the first cancer cells, second cancer cells, and/or stem cells are allogenic.

11. The composition according to any of claims 1 to 10, wherein the proliferation of the first cancer cells, second cancer cells, and stem cells is inhibited, in particular by radioactive irradiation or chemical cross-linking.

12. A pharmaceutical composition comprising a composition according to any one of claims 1 to 11, additionally comprising pharmaceutically acceptable diluents, carriers, excipients, fillers, binders, lubricants, glidants, disintegrants, adsorbents, and/or preservatives.

13. A composition according to any of claims 1 to 11 for use in medicine.

14. A composition according to any of claims 1 to 11 for use in a method of treatment, prevention and/or prevention of recurrence of cancer, particularly a cancer selected from the group consisting of gastrointestinal or colorectal tract, liver, pancreas, kidney, bladder, prostate, endometrium, head and neck cancer, ovary, testes, prostate, skin, eye, melanoma, invasive oral cancer, small cell and non-small cell lung cancer, hormone-dependent breast cancer, hormone independent breast cancer, transitional and squamous cell cancer, neurological malignancy, including neuroblastoma, endocrinological tumour, hematologic neoplasia, carcinoma in situ, hyperplastic lesion, adenoma, and fibroma, more particularly melanoma.

15. The composition for use according to claim 13 or 14, wherein the patient to be treated has a partially or completely different HLA type than the first cancer cells, second cancer cells, and/or stem cells, in particular a partially or completely different HLA type than the first cancer cells and the stem cells.
